# EUROPÄISCHE PATENTSCHRIFT

(11) **EP 0 741 686 B1**
(45) Veröffentlichungstag und Bekanntmachung des Hinweises auf die Patenterteilung: **22.04.1998**
(21) Anmeldenummer: 94905713.7
(22) Anmeldetag: 27.01.1994
(51) Int. Cl.: C07C 59/54, A61K 31/557, C07C 59/56, C07C 59/46, C07C 69/16, C07D 309/30

(54) **NEUE LEUKOTRIEN-B4-DERIVATE, VERFAHREN ZU IHRER HERSTELLUNG UND IHRE VERWENDUNG ALS ARZNEIMITTEL**
NEW LEUKOTRIENE B4 DERIVATIVES, METHODS OF PREPARING THEM AND THEIR USE AS DRUGS
NOUVEAUX DERIVES DES LEUCOTRIENES B4, LEURS PROCEDES DE FABRICATION ET LEUR UTILISATION COMME MEDICAMENTS

(43) Veröffentlichungstag der Anmeldung: 13.11.1996
(73) Patentinhaber: SCHERING AKTIENGESELLSCHAFT, 13342 Berlin (DE)
(72) Erfinder: SKUBALLA, Werner, D-13465 Berlin (DE); BUCHMANN, Bernd, D-10557 Berlin (DE); HEINDL, Josef, D-14129 Berlin (DE); FRÖHLICH, Wolfgang, D-10709 Berlin (DE); EKERDT, Roland, D-13369 Berlin (DE); GIESEN, Claudia, D-13587 Berlin (DE)
(86) Internationale Anmeldenummer: EP9400215
(87) Internationale Veröffentlichungsnummer: WO9520563

(56) Entgegenhaltungen:
- Keine einschlägigen Dokumente gefunden

## Beschreibung

Die Erfindung betrifft neue Leukotrien B₄-Derivaie, Verfahren zu ihrer Herstellung sowie ihre Verwendung als Arzneimittel. Die neuen Verbindungen sind optisch aktive Strukturanaloge von vorbekannten Leukotrien-B₄-Antagonisten, die einen Sechsring als Grundstrukturelement enthalten (DE-A 39 17 597, DE-A 42 27 790.6). Leukotrien B₄ (LTB₄) wurde von B. Samuelsson und Mitarbeiter als Metabolit der Arachidonsäure entdeckt. Bei der Biosynthese wird durch das Enzym 5-Lipoxygenase zunächst als zentrales Zwischenprodukt das Leukotrien A₄ gebildet, das dann durch eine spezifische Hydrolase in das LTB₄ umgewandelt wird.

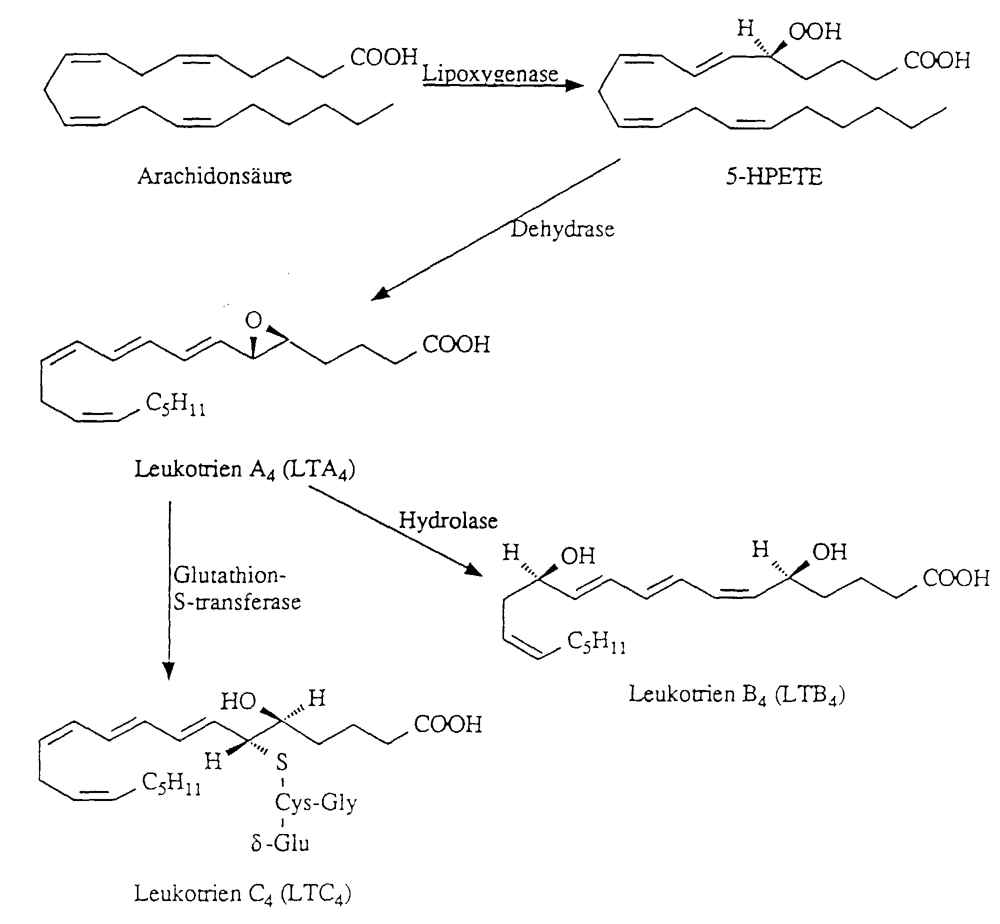

Die Nomenklatur der Leukotriene kann folgenden Arbeiten entnommen werden:
a) B. Samuelsson et al., Prostaglandins 19, 645 (1980); 17, 785 (1979).
b) C. N. Serhan et al., Prostaglandins 34, 201 (1987)

Die physiologische und insbesondere die pathophysiologische Bedeutung des Leukotrien B₄ wird in einigen neueren Arbeiten zusammengefaßt: a) The Leukotriens, Chemistry and Biology eds. L. W. Chakrin, D. M. Bailey, Academic Press 1984. b) J. W. Gillard et al., Drugs of the Future 12, 453 (1987). c) B. Samuelson ., Sciences 237, 1171 (1987). d) C. W. Parker, Drug Development Research 10, 277 (1987). Hieraus ergibt sich, daß LTB₄ ein wichtiger Entzündungsmediator füe entzündliche Erkrankungen ist, bei denen Leukozyten in das erkrankte Gewebe einwandern.
Die Effekte von LTB₄ werden auf zellulärer Ebene durch die Bindung von LTB₄ an einen spezifischen Rezeptor ausgelöst.
Von LTB₄ weiß man, daß es die Adhäsion von Leukozyten an die Blutgefäßwand verursacht. LTB₄ ist chemotaktisch wirksam, d. h. es löst eine gerichtete Wanderung von Leukozyten in Richtung eines Gradienten steigender Konzentration aus. Außerdem verändert es aufgrund seiner chemotaktischen Aktivität indirekt die vasculäre Permeabilität, wobei ein Synergismus mit Prostaglandin E₂ beobachtet wird. LTB₄ spielt offensichtlich bei entzündlichen, allergischen und immunologischen Prozessen eine entscheidende Rolle.

Leukotriene und insbesondere LTB₄ sind an Hauterkrankungen beteiligt, die mit entzündlichen Prozessen (erhöhte Gefäßpermeabilität und Ödembildung, Zellinfiltration), erhöhter Proliferation der Hautzellen und Juckreiz einhergehen, wie beispielsweise bei Ekzemen, Erythemen, Psoriasis, Pruritus und Akne. Pathologisch erhöhte Leukotrien-Konzentrationen sind an der Entstehung vieler Dermatiden entweder ursächlich beteiligt, oder es besteht ein Zusammenhang zwischen der Persistenz der Dermatiden und den Leukotrienen. Deutlich erhöhte Leukotrienkonzentrationen wurden beispielsweise in der Haut von Patienten mit Psoriasis oder atopischer Dermatitis gemessen.
Leukotriene und insbesondere LTB₄ sind auch an Erkrankungen innerer Organe beteiligt, für die eine akute oder chronische entzündliche Komponente beschrieben wurde, z. B.: Gelenkerkrankungen (Athritis); Erkrankungen des Respirationstraktes (Asthma, Rhinitis und Allergien); entzündliche Darmerkrankungen (Colitis); sowie Reperfusionsschäden (an Herz-, Darm- oder Nierengewebe), die durch zeitweisen krankhaften Verschluß von Blutgefäßen entstehen.
Weiterhin sind Leukotriene und insbesondere LTB₄ bei der Erkrankung an multipler Sklerose beteiligt und bei dem klinischen Erscheinungsbild des Schocks (ausgelöst durch Infektionen, Verbrennungen oder bei Komplikationen bei der Nierendialyse oder anderen extra besprochenen Perfusionstechniken).
Leukotriene und insbesondere LTB₄ haben weiterhin einen Einfluß auf die Bildung von weißen Blutkörperchen im Knochenmark, auf das Wachstum von glatten Muskelzellen, vom Keratinozyten und von B-Lymphozyten. LTB₄ ist daher bei Erkrankungen mit entzündlichen Prozessen und bei Erkrankungen mit pathologisch gesteigerter Bildung und Wachstum von Zellen beteiligt.
Erkrankungen mit diesem Erscheinungsbild stellen z. B. Leukemie oder Artherosklerose dar.

Durch die Antagonisierung der Effekte insbesondere von LTB₄ sind die Wirkstoffe und deren Darreichungsformen dieser Erfindung spezifische Heilmittel bei der Erkrankung von Mensch und Tier, bei denen insbesondere Leukotriene eine pathologische Rolle spielen.

Neben therapeutischen Möglichkeiten, die sich aus einer Antagonisierung der LTB₄-Wirkung mit LTB₄-Analoga ableiten lassen, konnte auch die Nützlichkeit und potentielle Anwendung von Leukotrien B₄-Agonisten zur Behandlung von Pilzerkrankungen der Haut gezeigt werden (H. Katayama, Prostaglandins 34, 797 (1988)).

Die Erfindung betrifft Leukotrien-B₄-Derivate der allgemeinen Formel I, 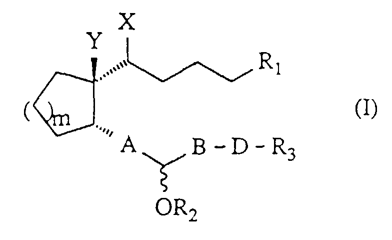 worin
- R₁: CH₂OH, CH₃, CF₃, COOR₄, CONR₅R₆, und
- R₂: H oder ein organischer Säurerest mit 1-15 C-Atomen darstellt,
- R₃: H, gegebenenfalls einfach oder mehrfach substituiertes C₁-C₁₄-Alkyl, C₃-C₁₀-Cycloalkyl, gegebenenfalls unabhängig voneinander einfach oder mehrfach durch Halogen, Phenyl, C₁-C₄-Alkyl, C₁-C₄-Alkoxy, Fluormethyl, Chlormethyl, Trifluormethyl, Carbonyl, Carboxyl oder Hydroxy substituierter C₆-C₁₀-Arylrest oder ein 5-6 gliedriger aromatischer heterocyclischer Ring mit wenigstens 1 Heteroatom symbolisieren,
- R₄: Wasserstoff, C₁-C₁₀ Alkyl, C₃-C₁₀ Cycloalkyl, gegebenenfalls durch 1-3 Halogen, Phenyl, C₁-C₄-Alkyl, C₁-C₄-Alkoxy, Fluormethyl, Chlormethyl, Trifluormethyl, Carboxyl oder Hydroxy substituierter C₆-C₁₀-Arylrest, CH₂-CO-(C₆-C₁₀) Aryl oder ein 5-6 gliedriger Ring mit wenigstens 1 Heteroatom bedeutet,
- A: eine trans, trans-CH=CH-CH=CH, eine -CH₂CH₂-CH=CH- oder eine Tetramethylengruppe,
- B: eine C₁-C₁₀- gerad- oder verzweigtkettige Alkylengruppe, die gegebenenfalls durch Fluor substituiert sein kann oder die Gruppe 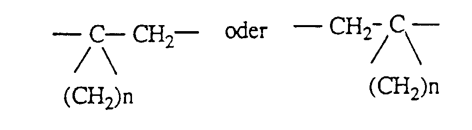 symbolisiert,
- D: eine Direktbindung, Sauerstoff, Schwefel, -C≡C-, -CH=CR₇, oder gemeinsam mit
- B: auch eine Direktbindung bedeuten können,
- R₅ und R₆: gleich oder verschieden sind und H oder gegebenenfalls durch Hydroxygruppen substituiertes C₁-C₄-Alkyl oder R₆ H und R₅ C₁-C₁₅-Alkanoyl oder R₈SO₂- darstellen, gegebenenfalls mit OH substituiert sind,
- R₇: H, C₁-C₅ Alkyl, Chlor, Brom bedeutet,
- R₈: die gleiche Bedeutung wie R₃ besitzt,
- m: 1-3 bedeutet
- n: 2-5 ist sowie, wenn R₄ Wasserstoff bedeutet, deren Salze mit physiologisch verträglichen Basen und deren Cyclodextrinclathrate.
- X und Y: eine direkte Bindung bedeutet, wobei das resultierende Olefin E oder Z konfiguriert sein kann oder X ein α- oder β-ständiges Fluoratom darstellt, und y ein β-ständiges Wasserstoffatom bedeutet.

Die Gruppe OR₂ kann α- oder β-ständig sein. Die Formel I umfaßt sowohl Racemate als auch die möglichen reinen Diastereomeren und Enantiomeren.

Als Alkylgruppen R₄ kommem gerad- oder verzweigtkettige Alkylgruppen mit 1-10 C-Atomen in Betracht, wie beispielsweise Methyl, Ethyl, Propyl, Butyl, Isobutyl, tert.-Butyl, Penty, Neopentyl, Hexyl, Heptyl, Decyl.
Die Alkylgruppen R₄ können gegbenenfalls ein- bis mehrfach substituiert sein durch Halogenatome, Alkoxygruppen, gegebenenfalls substituierte Aryl- bzw. Aroylgruppen mit 6-10 C-Atomen (Bezüglich möglicher Substituenten siehe unter Aryl R₄), Dia1kylamino und Trialkylammonium mit 1-4 C-Atomen im Alkyl-Teil, wobei die einfache Substitution bevorzugt sein soll. Als Substituenten seien beispielsweise genannt Fluor, Chlor oder Brom, Phenyl, Dimethylamino, Diethylamino, Methoxy, Ethoxy. Als bevorzugte Alkylgruppen R₄ sind solche mit 1-4 C-Atomen zu nennen.

Die Cycloalkylgruppe R₄ kann im Ring 3-10, vorzugsweise 5 und 6 Kohlenstoffatome enthalten. Die Ringe können durch Alkylgruppen mit 1-4 Kohlenstoffatomen substituiert sein. Beispielsweise seien genannt Cyclopentyl, Cyclohexyl, Methylcyclohexyl.

Als Arylgruppen R₄ kommen sowohl substituierte wie auch unsubstituierte Arylgruppen mit 6-10 C-Atomen in Betracht, wie beispielsweise Phenyl, 1-Naphthyl und 2-Naphthyl, die jeweils substituiert sein können durch 1-3 Halogenatome (F, Cl, Br), eine Phenylgruppe, 1-3 Alkylgruppen mit jeweils 1-4 C-Atomen, eine Chlormethyl-, eine Fluormethyl-, Trifluormethyl-, Carboxyl-, Hydroxy- oder Alkoxygruppe mit 1-4 C-Atomen. Bevorzugte Substituenten in 3- und 4-Stellung am Phenylring sind zum Beispiel Fluor, Chlor, Alkoxy oder Trifluormethyl, in 4-Stellung dagegen Hydroxy.

Als heterocyclische Gruppen R₄ kommen 5- und 6-gliedrige aromatische Heterocyclen in Frage, die wenigsten 1 Heteroatom, vorzugsweise Stickstoff, Sauerstoff oder Schwefel enthalten. Beispielsweise seien genannt 2-Furyl, 2-Thienyl, 2-pyridyl, 3-Pyridyl, 4-Pyridyl, Oxazolyl, Thiazolyl, Pyrimidinyl, Pyridazinyl, 3-Furyl, 3-Thienyl, 2-Tetrazolyl u. a.

Als Säurerest R₅ kommen solche physiologisch verträglicher Säuren in Frage. Bevorzugte Säuren sind organische Carbonsäuren und Sulfonsäuren mit 1-15 Kohlenstoffatomen, die der aliphatischen, cycloaliphatischen, aromatischen, aromatisch-aliphatischen und heterocyclischen Reihe angehören. Diese Säuren können gesättigt, ungesättigt und/oder mehrbasisch und/oder in üblicher Weise substituiert sein. Als Beispiele für die Substituenten seien C₁₋₄-Alkyl-, Hydroxy-, C₁₋₄-Alkoxy, Oxo- oder Aminogruppen oder Halogenatome (F, Cl, Br) erwähnt. Beispielweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diethylessigsäure, tert.-Butylessigsäure, Cyclopropylessigsäure, Cyclopentylessigsäure, Cyclohexylessigsäure, Cyclopropancarbonsäure, Cyclohexancarbonsäure, Phenylessigsäure, Phenoxyessigsäure, Methoxyessigsäure, Ethoxyessigsäure, Mono-, Di- und Trichloressigsäure, Aminoessigsäure, Diethylaminoessigsäure, Piperidinoessigsäure, Morpholinoessigsäure, Milchsäure, Bernsteinsäure, Adipinsäure, Benzoesäure, mit Halogen (F, Cl, Br) oder Trifluormethyl-, Hydroxy, C₁₋₄-Alkoxy- oder Carboxy-Gruppen substituierte Benzoesäuren, Nikotinsäure, Isonikotinsäure, Furan-2-carbonsäure, Cyclopentylpropionsäure. Als bevorzugte Arylsulfonylreste und Alkansulfonylreste R₈SO₂- werden solche betrachtet, die sich von einer Sulfonsäure mit bis zu 10 Kohlenstoffatomen ableiten. Als Sulfonsäuren kommen beispielsweise Methansulfonsäure, Ethansulfonsäure, Isopropansulfonsäure, β-Chlorethansulfonsäure, Butansulfonsäure, Cyclopentansulfonsäure, Cyclohexansulfonsäure, Benzolsulfonsäure, p-Toluolsulfonsäure, p-Chlorbenzolsulfonsäure, N,N-Dimethylaminosulfonsäure, N,N-Diethylaminosulfonsäure, N,N-Bis-(β-chlorethyl)-aminosulfonsäure, N,N-Diisobutylaminosulfonsäure, N,N-Dibutylaminosulfonsäure, Pyrrolidino-, Piperidino-, Piperazino-, N-Methylpiperazino- und Morpholinosulfonsäure in Frage.

Als Alkylgruppen R₃ kommen gerad- und verzweigtkettige, gesättigte und ungesättigte Alkylreste, vorzugsweise gesättigte, mit 1-14, insbesondere 1-10 C-Atomen, in Frage, die gegebenenfalls durch gegebenenfalls substituiertes Phenyl (Substitution s. unter Aryl R₄) substituiert sein können. Beispielsweise seien genannt Methy-, Ethyl-, Propyl-, Butyl-, Isobutyl-, tert.-Butyl-, Pentyl-, Hexyl-, Heptyl-, Octyl-, Butenyl-, Isobutenyl-, Propenyl-, Pentenyl-, Benzyl-, m- und p-Chlorbenzylgruppen. Sind die Alkylgruppen R₃ Halogen-substituiert, kommen als Halogene Fluor, Chlor und Brom in Frage.

Als Beispiele für Halogen-substituierte Alkylgruppen R₃ kommen Alkyle mit terminalen Trifluormethylgruppen in Betracht.

Die Cycloalkylgruppe R₃ kann im Ring 3-10, vorzugsweise 3-6 Kohlenstoffatome enthalten. Die Ringe können durch Alkylgruppen mit 1-4 Kohlenstoffatomen gegebenenfalls durch Halogene substituiert sein. Beispielsweise genannt seien Cyclopropyl, Cyclobutyl, Cyclopentyl, Cyclohexyl, Methyl-cyclohexyl, Fluor-cyclohexyl.

Als substituierte bzw. unsubstituierte Arylgruppen R₃ kommen beispielweise in Betracht: Phenyl, 1-Naphthyl und 2-Naphthyl, die jeweils substituiert sein können durch 1-3 Halogenatome (F, Cl, Br), eine Phenylgruppe, 1-3 Alkylgruppen mit jeweils 1-4 C-Atomen eine Chlormethyl-, Fluormethyl-, Trifluormethyl-, Carboxyl-, C₁-C₄ Alkoxy- oder Hydroxygruppe. Bevorzugt ist die Substitution in 3- und 4- Stellung an Phenylring zum Beispiel durch Fluor, Chlor, Alkoxy oder Trifluormethyl oder in 4-Stellung durch Hydroxy.

Als heterocyclische aromatische Gruppen R₃ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3- Furyl, 3-Thienyl, u. a.

Als Alkylengruppe B kommen geradkettige oder verzweigte, gesättigte oder ungesättigte Alkylenreste, vorzugsweise gesättigte mit 1-10, insbesondere mit 1-5 C-Atomen, in Frage, die gegebenenfalls durch Fluoratome substituiert sein können. Beispielsweise seien genannt: Methylen, Fluormethylen, Difluormethylen, Ethylen, 1,2-Propylen, Ethylethylen, Trimethylen, Tetramethylen, Pentamethylen, 1,2-Difluorethylen, 1-Fluorethylen, 1-Methyltetramethylen, 1-Methyl-tri-methylen, 1-Methylen-ethylen, 1-Methylen-tetramethylen.
Die Alkylengruppe B kann weiterhin die Gruppe 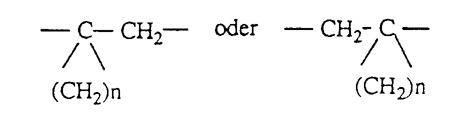 darstellen, wobei n=2-5, bevorzugt 3-5 bedeutet.

Als Säurereste R₂ kommen solche von physiologisch verträglichen Säureresten in Frage. Bevorzugte Säuren sind organische Carbonsäuren und Sulfonsäuren mit 1-15 Kohlenstoffatomen, die der aliphatischen, cyclo-aliphatischen, aromatischen, aromatisch-aliphatischen oder heterocyclischen Reihe angehören. Diese Säuren können gesättigt, ungesättigt und/oder mehrbasisch und/oder in üblicher Weise substituiert sein. Als Beispiele für die Substituenten seien C₁₋₄-Alky, Hydroxy, C₁₋₄-Alkoxy, Oxo- oder Aminogruppen oder Halogenatome (F, Cl, Br) erwähnt. Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diethylessigsäure, tert.-Butylessigsäure, Cyclopentylessigsäure, Cyclohexylessigsäure, Cyclohexancarbonsäure, Phenylessigsäure, Phenoxyessigsäure, Methoxyessigsäure, Ethoxyessigsäure, Mono-, Di- und Trichloressigsäure, Aminoessigsäure, Diethylaminoessigsäure, Piperidinoessigsäure, Morpholinoessigsäure, Milchsäure, Bernsteinsäure, Adipinsäure, Benzoesäure, mit Halogen (F, Cl, Br) oder Trifluormethyl-, Hydroxy, C₁₋₄-Alkoxy- oder Carboxy-Gruppen substituierte Benzoesäuren, Nikotinsäure, Isonikotinsäure, Furan-2-carbonsäure, Cyclopentylpropionsäure. Als bevorzugte Säurereste R₂ und R₃ werden solche Acylreste mit bis zu 10 Kohlenstoffatomen betrachtet.

Die Alkyreste R₅ und R₆, die gegebenenfalls Hydroxygruppen enthalten, sind geradkettige oder verzweigte Alkylreste, insbesondere geradkettige wie zum Beispiel Methyl, Ethyl, Propyl, Butyl, Pentyl, Hexyl, besonders bevorzugt Methyl.

R₇ als C₁₋₅-Alkyl bedeutet geradkettige oder verzweigtkettige Alkylreste wie sie für R₃ bzw. R₄ bereits genannt wurden. Bevorzugte Alkylreste R₇ sind Methyl, Ethyl, Propyl und Isopropyl.

Zur Salzbildung sind anorganische und organische Basen geeignet, wie sie dem Fachmann zur Bildung physiologisch verträglicher Salze bekannt sind. Beispielsweise seien genannt Alkalihydroxiden wie Natrium- und Kaliumhydroxid, Erdalkalihydoxide wie Calciumhydoxid, Ammoniak, Amine, wie Ethanolamin, Diethanolamin, Triethanolamin, N-Methylglucamin, Morpholin, Tris-(hydroxymethyl)-methylamin usw.

Um zu den Cyclodextrinclathraten zu gelangen werden die Verbindungen der Formel I mit α-, β- oder γ-Cyclodextrin umgesetzt. Bevorzugt sind β-Cyclodextrinderivate.

Bevorzugte Verbindungen der vorliegenden Erfindung sind Verbindungen der allgemeinen Formel I, wobei die Reste die folgende Bedeutung haben:
- R₁: ist CH₂OH, CONR₅R₆, COOR₄ mit R₄ in der Bedeutung eines Wasserstoffatoms, eines Alkylrestes mit 1-10 C-Atomen, eines Cycloalkylrestes mit 5-6 C-Atomen, eines gegebenenfalls durch 1-2 Chlor, Brom, Phenyl, C₁₋₄-Alkyl, C₁₋₄-Alkoxy, Chlormethyl, Fluormethyl, Trifluormethyl, Carboxy oder Hydroxy substituierten Phenylrestes
- m: ist 1-3
- A: ist eine transtrans-CH=CH-CH=CH- oder Tetramethylengruppe;
- B: ist eine geradkettige oder verzweigtkettige gesättigte oder ungesättigte Alkylengruppe mit bis zu 10 C-Atomen, die gegebenenfalls durch Fluor substituiert sein kann, oder die Gruppe 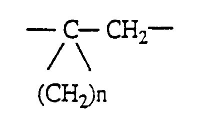 mit n=2-5;
- D: ist eine Direktverbindung, Sauerstoff, Schwefel, eine -C≡C-Gruppe oder eine -CH=CR₇-Gruppe mit R₇ als Wasserstoff, C₁₋₅-Alkyl, Chlor oder Brom; oder
- B und D: sind gemeinsam eine Direktverbindung;
- R₂: bedeutet Wasserstoff oder einen organischen Säurerest mit 1-15 C-Atomen;
- R₅ und R₆: die oben angegebenen Bedeutungen aufweisen;
- R₃: ist ein Wasserstoffatom, C₁₋₁₀-Alkyl, Cycloalkyl mit 5-6 C-Atomen, ein gegebenenfalls durch 1-2 Chlor, Brom, Phenyl, C₁₋₄-Alkyl, C₁₋₄-Alkoxy, Chlormethyl, Fluormethyl, Trifluormethyl, Carboxy oder Hydroxy substituierter Phenylrest und falls
- R₄: ein Wasserstoff bedeutet, deren Salze mit physiologisch verträglichen Basen und Cyclodextrinclathrate.

Besonders bevorzugte Verbindungen der vorliegenden Erfindung sind Verbindungen der allgemeinen Formel I, wobei die Reste folgende Bedeutung haben:
- R₁: ist CH₂OH, CONR₅R₆, COOR₄ mit R₄ in der Bedeutung eines Wasserstoffatoms, eines Alkylrestes mit 1-4 C-Atomen
- R₂: bedeutet Wasserstoff oder einen organischen Säurerest mit 1-6 C-Atomen;
- R₃: ist ein Wasserstoffatom oder C₁₋₁₀-Alkyl;
- R₅ und R₆: die oben angegebenen Bedeutungen aufweisen;
- A: ist eine trans, trans-CH=CH-CH=CH- oder Tetramethylengruppe;
- B: ist eine geradkettige oder verzweigtkettige Alkylengruppe mit bis zu 5 C-Atomen;
- D: ist eine Direktverbindung oder eine -C≡C-Gruppe oder eine -CH=CR₇-Gruppe mit R₇ als Wasserstoff oder C₁₋₅-Alkyl;
- B und D: sind gemeinsam eine Direktbindung;
und falls R₄ ein Wasserstoffatom bedeutet, deren Salze mit physiologisch verträglichen Basen und deren Cyclodextrinclathrate.

Außerdem betrifft die Erfindung ein Verfahren zur Herstellung der erfindungsgemäßen Verbindungen der allgemeinen Formel I, das dadurch gekennzeichnet ist, daß man einen Alkohol der Formel II, oder einen intermediären Sulfonsäurester, 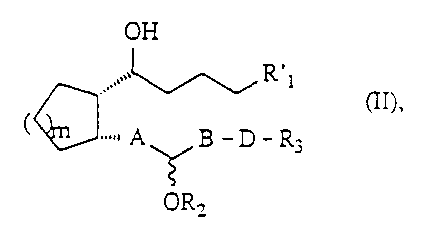 worin A, B, D, R₁, R₂ und R₃ die oben angegebene Bedeutung haben und R'₁ die gleiche Bedeutung wie R₁ besitzt oder die Gruppierung -CH₂OR₉ darstellt, worin R₉ einen leicht abspaltbaren Etherrest bedeutet, gegebenenfalls nach Schutz freier Hydroxygruppen in R₂ mit einem Wasserabspaltungsreagenz oder Fluorierungsreagenz der allgemeinen Formel III,

F₃SN(Alk)₃ (III)

worin Alk -CH₃ oder -CH₂CH₃ darstellt, gegebenenfalls in Gegenwart einer Base umsetzt und gegebenenfalls anschließend in beliebiger Reihenfolge Isomere trennt, geschützte Hydroxygruppen freisetzt und/oder eine freie Hydroxygruppe verestert und/oder die 1-Hydroxygruppe zur Carbonsäure oxidiert und/oder Doppelbindungen hydriert und/oder eine veresterte Carboxylgruppe verseift und/oder reduziert und/oder eine Carboxylgruppe verestert und/oder eine freie Carboxylgruppe in ein Amid überführt oder eine Carboxylgruppe mit einer physiologisch verträglichen Base in ein Salz überführt.

Als Etherreste R₉ in der Verbindung der Formel II kommen die dem Fachmann geläufigen Reste in Betracht. Bevorzugt sind leicht abspaltbare Etherreste, wie beispielsweise Dimethyl-tert.-butylsilyl, Trimethylsilyl-, Tribenzylsilyl-, Diphenyl-tert.-butylsilyl-, Tetrahydropyranyl-, Tetrahydrofuranyl, und α-Ethoxyethyl, um nur einige zu nennen.

Die Umsetzung der Verbindung der allgemeinen Formel II mit einem Fluorierungsreagenz der allgemeinen Formel III wird bei Temperaturen von -100°C bis 100°C, vorzugsweise -78°C bis 80°C in einem aprotischen Lösungsmittel oder Lösungsmittelgemisch, beispielsweise Tetrahydrofuran, Diethylether, gegebenenfalls in Gegenwart eines Amins vorgenommen. Als Amine kommen beispielsweise Triethylamin, Dimethylaminopyridin oder Pyridin in Frage. Bei dieser Unsetzung erhält man neben der 5-Fluorverbindung auch das Δ^{5,6}-Olefin, welches chromatographisch abgetrennt werden kann.
Will man nur das Δ^{5,6}-Olefin erhalten, kann man die Hydroxygruppe, gegebenenfalls über einen intermediären Sulfonsäurrester, abspalten. Als Wasserabspaltungsreagenz kommt beispielsweise das sogenannte Burgess-Reagenz (J. Am. Chem. Soc. 90, 4744 (1968)) in Frage.
Die Umsetzung der Verbindung der allgemeinen Formel II zu einem intermediären 9-Sulfonsäureester erfolgt in an sich bekannter Weise mit einem Alkyl- oder Arylsulfonylchlorid bzw. Alkyl- oder Arylsulfonylanhydrid in Gegenwart eines Amins wie beispielsweise Pyridin, Triethylamin oder DMAP bei Temperaturen zwischen -60°C und +100°C, vorzugsweise -20°C bis +50°C. Die Eliminierung des 5-Sulfonats erfolgt mit einer Base, vorzugsweise Kalium-tert-butylat, 1,5-Diazabicyclo[4.3.0]non-5-en oder 1,8-Diazabicyclo[5.4.0]undec-7-en in einem inerten Lösungsmittel wie beispielsweise Dimethylformamid, Dimethylacetamid, Dimethylsulfoxid, Dimethoxyethan, Tetrahydrofuran usw. bei Temperaturen zwischen 0°C und 100°C, vorzugsweise 20°C bis 80°C.

Die Reduktion zu den Verbindungen der Formel I mit R₁ in der Bedeutung einer CH₂OH-Gruppe wird mit einem für die Reduktion von Estern oder Carbonsäuren geeigneten Reduktionsmittel wie beispielsweise Lithiumaluminiumhydrid, Diisobutylaluminiumhydrid usw. durchgeführt. Als Lösungsmittel kommen Diethylether, Tetrahydrofuran, Dimethoxyethan, Toluol usw. in Frage. Die Reduktion wird bei Temperaturen von -30°C bis zur Siedetemperatur des verwendeten Lösungsmittels, vorzugsweise 0°C bis 30°C vorgenommen.

Die Veresterung der Alkohole der Formel I (R₂=H) erfolgt in an sich bekannter Weise. Zum Beispiel erfolgt die Veresterung dadurch, daß man ein Säurederivat, vorzugsweise ein Säurehalogenid oder Säureanhydrid, in Gegenwart einer Base wie beispielsweise NaH, Pyridin, Triethylamin, Tributylamin oder 4-Dimethylaminopyridin mit einem Alkohol der Formel I umsetzt. Die Umsetzung kann ohne Lösungsmittel oder in einem inerten Lösungsmittel, vorzugsweise Aceton, Acetonitril, Dimethylacetamid, Dimethylsulfoxid bei Temperaturen über oder unter Raumtemperatur, zum Beispiel zwischen -80°C bis 100°C, vorzugsweise bei Raumtemperatur, vorgenommen werden.

Die Oxidation der 1-Hydroxygruppe wird nach den dem Fachmann bekannten Methoden vorgenommen. Als Oxidationsmittel können beispielsweise dienen: Pyridiniumdichromat (Tetrahedron Letters, 1979, 399), Jones Reagenz (J. Chem. Soc. 1953, 2555) oder Platin/Sauerstoff (Adv. in Carbohydrate Chem. 17, 169 (1962) oder Collins-Oxidation (Tetrahedron Letters 1968, 3363 und anschließende Jones-Oxidation. Die Oxidation mit Pyridiniumdichromat wird bei Temperaturen von 0°C bis 100°C vorzugsweise bei 20°C bis 40°C in einem gegen das Oxidationsmittel inerten Lösungsmittel, beispielsweise Dimethylformamid, durchgeführt.

Die Oxidation mit Jones-Reagenz wird bei Temperaturen von -40°C bis +40°C, vorzugsweise 0°C bis 30°C in Aceton als Lösungsmittel ausgeführt.

Die Oxidation mit Platin/Sauerstoff wird bei Temperaturen von 0°C bis 60°C, vorzugsweise 20°C bis 40°C in einem gegen das Oxidationsmittel inerten Lösungsmittel, wie z. B. Essigester, durchgeführt

Die Verseifung der Ester der Formel I wird nach den dem Fachmann bekannten Methoden durchgeführt, wie beispielsweise mit basischen Katalysatoren. Die Verbindungen der Formel I können durch die üblichen Trennmethoden in die optischen Isomeren getrennt werden (Asymmetric Synthesis, Vol. 1-5, Ed. J. D. Morrison, Academic Press. Inc. Orlando etc., 1985; Chiral Separarations by HPLC, Ed. A. M. Krstulovic; John Wiley & Sons; New York etc. 1989).

Die Freisetzung der funktionell abgewandelten Hydroxygruppen erfolgt nach bekannten Methoden. Beispielsweise wird die Abspaltung von Hydroxyschutzgruppen, wie beispielsweise des Tetrahydropyranylrestes, in einer wässrigen Lösung einer organischen Säure, wie z. B. Oxalsäure, Essigsäure, Propionsäure u. a., oder in einer wässrigen Lösung einer anorganischen Säure, wie z. B. Salzsäure durchgeführt. Zur Verbesserung der Löslichkeit wird zweckmäßigerweise ein mit Wasser mischbares inertes organisches Lösungsmittel zugesezt Geeignete organische Lösungsmittel sind z. B. Alkohole, wie Methanol und Ethanol und Ether, wie Dimethoxyethan, Dioxan und Tetrahydrofuran. Tetrahydrofuran wird bevorzugt angewendet. Die Abspaltung wird vorzugsweise bei Temperaturen zwischen 20°C und 80°C durchgeführt Die Abspaltung der Silyletherschutzgruppen erfolgt beispielsweise mit Tetrabutylammoniumfluorid oder mit Kaliumfluorid in Gegenwart eines Kronenethers (wie zum Beispiel Dibenzo[18]-Krone-6). Als Lösungsmittel sind beispielsweise geeignet Tetrahydrofuran, Diethylether, Dioxan, Dichlormethan, usw. Die Abspaltung wird vorzugsweise bei Temperaturen zwischen 0°C und 80°C durchgeführt.

Die Verseifung der Acylgruppen erfolgt beispielsweise mit Alkali- oder Erdalkalicarbonaten oder -hydroxiden in einem Alkohol oder in der wässrigen Lösung eines Alkohols. Als Alkohol kommen niedere aliphatische Alkohole in Betracht, wie z. B. Methanol, Ethanol, Butanol usw., vorzugsweise Methanol. Als Alkalicarbonate und -hydroxide seien Kalium- und Natriumsalze genannt. Bevorzugt sind Kaliumsalze.

Als Erdalkalicarbonate und -hydroxide sind beispielsweise geeignet Calciumcarbonat, Calciumhydroxid und Bariumcarbonat. Die Umsetzung erfolgt bei -10°C bis +70°C, vorzugsweise bei +25°C.

Die Einführung der Estergruppe -COOR₄ für R₁, bei welcher R₄ eine Alkylgruppe mit 1-10 C-Atomen darstellt, erfolgt nach den dem Fachmann bekannten Methoden. Die 1-Carboxy-verbindungen werden beispielsweise mit Diazokohlenwasserstoffen in an sich bekannter Weise umgesetzt. Die Veresterung mit Diazokohlenwasserstoffen erfolgt z. B. dadurch, daß man eine Lösung des Diazokohlenwasserstoffs in einem inerten Lösungsmittel, vorzugsweise in Diethylether, mit der 1-Carboxyverbindung in dem gleichen oder in einem anderen inerten Lösungsmittel, wie z. B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seiten 389-394 (1954)].

Die Einführung der Estergruppe -COOR₄ für R₁, bei welcher R₄ eine substituierte oder unsubstituierte Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base, beispielsweise Pyridin, Dimethylaminopyridin, Triethylamin, in einem inerten Lösungsmittel umgesetzt. Als Lösungsmittel kommen Methylenchlorid, Ethylenchlorid, Chloroform, Essigester, Tetrahydrofuran, vorzugsweise Chloroform in Frage. Die Reaktion wird bei Temperaturen zwischen -30°C und +50°C, vorzugsweise bei 10°C durchgeführt.

Sollen im Primärprodukt enthaltene C=C-Doppelbindungen reduziert werden, erfolgt die Hydrierung nach an sich bekannten Methoden.

Die Hydrierung des Δ^{8.10}-Diensystems wird in an sich bekannter Weise bei tiefen Temperaturen, vorzugsweise bei etwa -20°C bis +30°C in einer Wasserstoffatmosphäre in Gegenwart eines Edelmetallkatalysators durchgeführt. Als Katalysator ist zum Beispiel 10% Palladium auf Kohle geeignet.

Die Leukotrien-B₄-Derivate der Formel I mit R₄ in der Bedeutung eines Wasserstoffs können mit geeigneten Mengen der entsprechenden anorganischen Basen unter Neutralisierung in ein Salz überführt werden. Beispielsweise erhält man beim Lösen der entsprechenden Säuren in Wasser, das die stöchiometrische Menge der Base enthält, nach Abdampfen des Wassers oder nach Zugabe eines mit Wasser mischbaren Lösungsmittels, z. B. Alkohol oder Aceton, das feste anorganische Salz.

Zur Herstellung eines Aminsalzes wird LTB₄-Säure z. B. in einem geeigneten Lösungsmittel, beispielsweise Ethanol, Aceton, Diethylether, Acetonitril oder Benzol gelöst und mindestens die stöchiometrische Menge des Amins der Lösung zugesezt. Dabei fällt das Salz gewöhnlich in fester Form an oder wird nach Verdampfen des Lösungsmittels in üblicher Weise isoliert.

Die Einführung der Amidgruppe -CONHR₅ mit R₅ in der Bedeutung von Alkanoyl erfolgt nach den dem Fachmann bekannten Methoden. Die Carbonsäuren der Formel I (R₄=H), werden zunächst in Gegenwart eines tertiären Amins, wie beispielsweise Triethylamin, mit Chlorameisensäurebutylester in das gemischte Anhydrid überführt. Die Umsetzung des gemischten Anhydrids mit dem Alkalisalz des entsprechenden Amids oder mit Ammoniak (R₅=H) erfolgt in einem inerten Lösungsmittel oder Lösungsmittelgemisch, wie beispielsweise Tetrahydrofuran, Dimethoxyethan, Dimethylformanid, Hexamethylphosphorsäuretriamid, bei Temperaturen zwischen -30°C und +60°C, vorzugsweise bei 0°C bis 30°C. Eine weitere Herstellungsart für die Amide besteht in der Amidolyse des 1-Esters (R₁=COOR₄) mit dem entsprechenden Amin.

Eine weitere Möglichkeit für die Einführung der Amidgruppe -CONHR₅ besteht in der Umsetzung einer 1-Carbonsäure der Formel I (R₄=H), in der freie Hydroxygruppen gegebenenfalls intermediär geschützt sind, mit Verbindungen der Formel IV,

O=C=N-R₅ (IV)

worin R₅ die oben angegebene Bedeutung hat.

Die Umsetzung der Verbindung der Formel I (R₄=H) mit einem Isocyanat der Formel IV erfolgt gegebenenfalls unter Zusatz eines tertiären Amins, wie z. B. Triethylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diethylether, Toluol, bei Temperaturen zwischen -80°C bis 100°C, vorzugsweise bei 0°C bis 30°C, vorgenommen werden.

Zur Herstellung der übrigen Amide kann man beispielsweise die gewünschten Säureanhydride mit Ammoniak oder den entsprechenden Aminen umsetzen.

Enthält das Ausgangsprodukt OH-Gruppen im Leukotrien-B₄-Rest, so werden diese OH-Gruppen auch zur Reaktion gebracht. Werden letztlich Endprodukte gewünscht, die freie Hydroxylgruppen enthalten, geht man zweckmäßigerweise von Ausgangsprodukten aus, in denen diese durch vorzugsweise leicht abspaltbare Ether- oder Acylreste intermediär geschützt sind.

Die Trennung von Diastereomeren erfolgt nach den dem Fachmann bekannten Methoden, beispielsweise durch Säulenchromatographie.

Die als Ausgangsmaterial dienenden Verbindungen der Formel II sind in der DE-A 42 27 790.6 beschrieben oder können beispielsweise hergestellt werden, indem man in an sich bekannter Weise cis-1,2-Diacetoxymethyl-cyclohex-4-en oder cis-1,2-Diacetoxymethyl-cyclohexan oder cis-1,2-Diacetoxymethyl cyclopentan oder cis-1,2-Diacetoxymethyl cycloheptan mit einer Lipase enantioselektiv hydrolysiert (J. B. Jones et al., J. Chem. Soc. Chem. Commun. 1985, 1563; M. Schneider et al., Tetrahedron Lett. 26, 2073 (1985); H. J. Gais et al., Tetrahedron Lett 28, 3471 (1987)). Das auf diese Weise hergestellte optisch aktive Monoacetat wird anschließend in den Tert.butyldimethylsilylether überführt, gegebenenfalls hydriert und anschließend mit Diisobutylaluminiumhydrid in den Monosilylether der Formel V 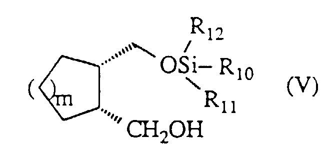 worin R₁₀, R₁₁ und R₁₂ gleich oder verschieden sind und C₁-C₄-Alkyl oder Phenyl bedeuten, überführt.

Durch die Oxidation z. B. mit Collins-Reagenz oder durch das Swern-Verfahren (Tetrahedron Letters 34, 1651 (1978)) wird der Aldehyd der Formel VI erhalten, 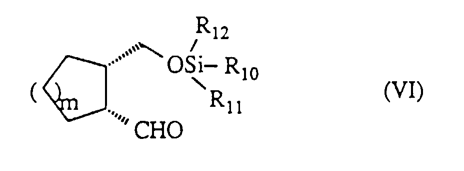 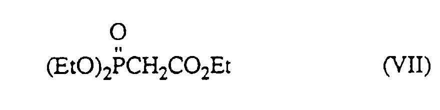 oder 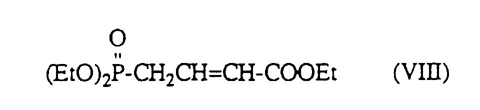 der in einer Wittig-Horner-Olefinierung mit dem Phosphonat der Formel VII und einer Base und gegebenenfalls anschließender Hydrierung sowie anschließender Reduktion der Estergruppe, Oxidation des primären Alkohols, nochmaliger Wittig-Horner-Olefinierung mit dem Phosphonat der Formel VII und gegebenenfalls anschliessender Hydrierung in den Ester der Formel IX oder einer Wittig-Horner Reaktion des Aldehyds der Formel VI mit einem Phosphonat der Formel VIII überführt wird, wobei A die 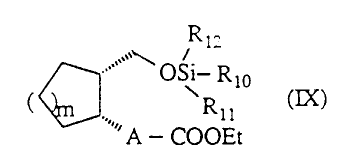 oben angegebene Bedeutung besitzt. Als Basen kommen beispielsweise Kaliumtert.-butylat, Diazabicyclononan, Diazabicycloundecan oder Natriumhydrid in Frage. Reduktion der Estergruppe, beispielsweise mit Diisobutylaluminiumhydrid und anschließende Oxidation des erhaltenen primären Alkohols z. B. mit Braunstein oder Collinsreagenz führt zum Aldehyd der Formel X 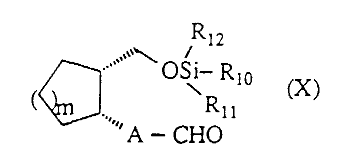 Die metallorganische Umsetzung des Aldehyds der Formel X mit einem Grignardreagenz der Formel XI, worin B, D

X-Mg-B-D-R₃ (XI)

und R₃ die oben angegebenen Bedeutungen aufweisen und X Chlor, Brom oder Iod bedeutet, führt nach Schutz der Hydroxygruppen (beispielsweise durch Acylierung) und gegebenenfalls Diastereomerentrennung zu den Verbindungen der Formel XII

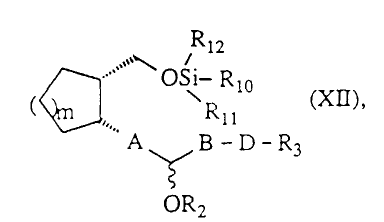

Die Herstellung der für die metallorganische Unsetzung benötigten Verbindung der Formel XI erfolgt durch Reaktion des entsprechenden terminalen Halogenids mit Magnesium. Durch Umsetzung des Silylethers XII mit Tetrabutylammoniumfluorid und gegebenenfalls Diastereomerentrennung wird der Alkohol der Formel XIII erhalten.

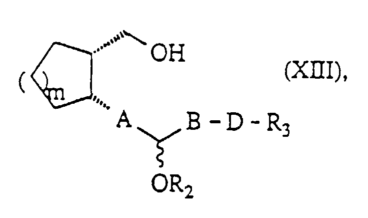

Zu den Verbindungen der Formel XII, worin B eine CH₂-Gruppe und D eine -C≡C-Gruppe oder eine CH=CR₇-Gruppe bedeutet, kann man gelangen, beispielsweise durch eine metallorganische Umsetzung eines Propargylhalogenids und anschließende Alkylierung mit einem entsprechenden Alkylhalogenid und gegebenenfalls anschliessende Lindlar-Hydrierung.

Ein alternativer Aufbau der unteren Kette geht von dem Aldehyd der Formel XIV aus, der aus der Wittig-Horner-Umsetzung des Aldehyds VI und anschließender Reduktion und Oxidation resultierte. 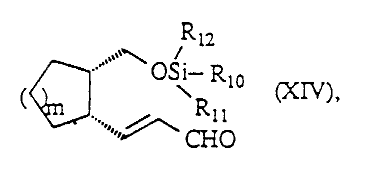 Wittig-Horner-Olefinierung des Aldehyds XIII mit einem Phosphonat der Formel XV 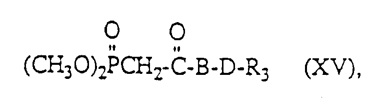 und Reduktion des entstandenen Ketons führte dann zum Alkohol der Formel XII und nach Acylierung und Silyletherspaltung zum Alkohol der Formel XIII, der gegebenenfalls in die Diastereomeren getrennt werden kann.

Die Verbindungen der allgemeinen Formel XIII sind in der DE-A 42 27 790.6 beschrieben oder können gemäß des in DE-A 42 27 790.6 vorgestellten Verfahrens hergestellt werden.

Die Oxidation der primären Alkoholgruppe in XIII z. B. mit Collinsreagenz oder Pyridiniumdichromat oder mit der Swernmethode führt zum Aldehyd der Formel XVI

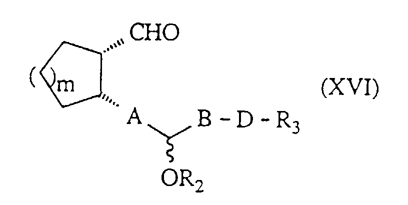

Die Umsetzung des Aldehyds der allgemeinen Formel XVI mit einer magnesiumorganischen Verbindung der Formel

HalMg-CH₂-CH₂-CH₂-R'₁ (XVII)

worin Hal Chlor, Brom oder Iod und R'₁ -CH₃, CF₃, oder -CH₂OR₉ darstellt, worin R₉ einen leicht abspaltbaren Etherrest bedeutet, führt zum Alkohol der allgemeinen Formel II. Anschließend kann man gegebenenfalls in beliebiger Reihenfolge Isomere trennen, geschützte Hydroxygruppen freisetzen und/oder eine freie Hydroxygruppe zur Carbonsäure oxidieren und/oder Doppelbindungen hydrieren und/oder eine veresterte Carboxylgruppe (R₁ = COOR₅) verseifen und/oder eine Carboxylgruppe (R₅ = H) verstern und/oder eine freie Carboxylgruppe (R₅ = H) in ein Amid (R₁ = CONR₆R₇) überführen oder eine Carboxylgruppe mit einer physiologisch verträglichen Base in ein Salz überführen.

Als Etherreste R₉ in der Verbindung der Formel II kommen die dem Fachmann geläufigen Reste in Betracht. Bevorzugt sind leicht abspaltbare Etherreste, wie beispielsweise Dimethyl-tert.-butylsilyl-, Trimethylsily-, Tribenzylsilyl-, Diphenyl-tert.butylsilyl-, Tetrahydropyranyl-, Tetrahydrofuranyl-, und α-Ethoxyethyl, um nur einige zu nennen.

Die Umsetzung der Verbindung der Formel II mit einer metallorganischen Verbindung der Formel XVII erfolgt in an sich bekannter Weise in einem inerten Lösungsmittel oder Lösungsmittelgemisch, wie zum Beispiel Dioxan, Toluol, Dimethoxyethan oder vorzugsweise Diethylether oder Tetrahydrofuran. Die Reaktion wird bei Temperaturen zwischen -100°C und 60°C, vorzugsweise bei -78°C bis 0°C durchgeführt.

Die Herstellung der für diese Umsetzung benötigten Verbindung XVII erfolgt durch Reaktion des entsprechenden durch eine leicht abspaltbare Ethergruppe geschützten Hydroxyhalogenids und anschließende Umsetzung mit Magnesium.

Der Einbau der chemisch und metabolisch labilen cis-Δ^{6,7}-Doppelbindung des LTB₄ in einen cis-1,2-substituierten Cycloalkylring führt zu einer Stabilisierung, wobei insbesondere durch weitere Derivatisierung der funktionellen Gruppen und/oder strukturelle Veränderungen der unteren Seitenkette, LTB₄-Derivate erhalten wurden, die als LTB₄-Antagonisten wirken können (DE-A 39 17 597 und DE-A 42 27 790.6 und DE-A 41 08 351 und 41 39 886.8).

Es wurde nun gefunden, daß durch Substitution der 5-Hydroxygruppe durch ein Fluoratom oder durch Einführung einer Doppelbindung in der 5,6-Position (Zählweise beginnend beim Caboxyl-C-Atom mit 1) und Weglassen der Hydroxygruppe in der 5-Position in derartigen Leukotrien B₄-Derivaten eine längere Wirkungsdauer, größere Selektivität und bessere Wirksamkeit erzielt werden kann.

Die Verbindungen der Formel I wirken antientzündlich, antiallergetisch und antiproliferativ. Daneben besitzen sie antimykotische Eigenschaften. Folglich stellen die neuen Leukotrien-B₄-Derivate der Formel I wertvolle pharmazeutische Wirkstoffe dar. Die Verbindungen der Formel I sind besonders zur topischen Applikation geeignet, da sie eine Dissoziation zwischen erwünschter topischer Wirksamkeit und unerwünschten systemischen Nebenwirkungen aufweisen.

Die neuen Leukotrien-B₄-Derivate der Formel I eignen sich in Kombination mit den in der galenischen Pharmazie üblichen Hilfs- und Trägermitteln zur lokalen Behandlung von Erkrankungen der Haut, bei denen Leukotriene eine wichtige Rolle spielen, z. B.: Kontaktdermatitis, Ekzemen der verschiedensten Art, Neurodermatosen, Erythrodermie, Pruritis vulvae et ani, Rosacea, Erythematodes cutaneus, Psoriasis, Lichen ruber planus et verrucosus und ähnlichen Hauterkrankungen.
Außerdem sind die neuen Leukotrien-B₄-Antagonisten zur Behandlung der multiplen Sklerose und der Symptome des Schocks geeignet.

Die Herstellung der Arzneimittelspezialitäten erfolgt in üblicher Weise, indem man die Wirkstoffe mit geeigneten Zusätzen in die gewünschte Applikationsform, wie zum Beispiel: Lösungen, Salben, Cremes oder Pflaster, überführt.

In den so formulierten Arzneimitteln ist die Wirkstoffkonzentration von der Applikationsform abhängig. Bei Lotionen und Salben wird vorzugsweise eine Wirkstoffkonzentration von 0,0001% bis 3% verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Leukotrien-B₄-Derivate auch in Form von Kapseln, Tabletten oder Dragees, die vorzugsweise 0,1 bis 100 mg Wirkstoff enthalten und oral appliziert werden oder in Form von Suspensionen, die vorzugseise 1-200 mg Wirkstoff pro Dosiseinheit enthalten und rektal appliziert werden auch zur Behandlung von Erkrankungen innerer Organe, bei denen Leukotriene eine wichtige Rolle spielen, wie z. B.: allergische Erkrankungen des Darmtraktes, wie der Colitis ulcerosa und der Colitis granulomatosa.

In diesen neuen Applikationformen eignen sich die neuen LTB₄-Derivate neben der Behandlung von Erkrankungen innerer Organe mit entzündlichen Prozessen auch zur Behandlung von Erkrankungen bei denen leukotrienabhängig das gesteigerte Wachstum und die Neubildung von Zellen im Vordergrund stehen. Beispiele sind Leukämie (gesteigertes Wachstum weißer Blutkörperchen) oder Artherosklerose (gesteigertes Wachstum glatter Muskelzellen von Blutgefäßen).

Die neuen Leukotrien-B₄-Derivate können auch in Kombination, wie z. B. mit Lipoxygenasehemmern, Cyclooxygenasehemmern, Glukokortikoiden, Prostacyclinagonisten, Thromboxanantagonisten, Leukotrien D₄-Antagonisten, Leukotrien E₄-Antagonisten, Leukotrien-F₄-Antagonisten, Phosphodiesterasehemmern, Calciumantagonisten, PAF-Antagonisten oder anderen bekannten Therapieformen der jeweiligen Erkrankungen verwendet werden.

Die nachfolgenden Ausführungsbeispiele dienen zur näheren Erläuterung des erfindungsgemäßen Verfahrens. In den Beispielen wurden nicht näher charakterisierte Diastereoisomere in der 12-Position als polar beziehungsweise unpolar charakterisiert (z. B. Diastereomer unpol (12)).

### Beispiel 1

### 5-[(E)-(2S)-2-((1E,3E)-(5S)-5-Hydroxy-6,6-trimethylen-9-phenyl-1,3-nonadien-8-inyl)-cyclohexyliden]-pentansäure Diastereomer pol (12)

Zu einer Lösung von 1,82 g (5S)-5-Hydroxy-5-[cis-(2S)-2-((1E,3E)-(5S)-5-acetoxy-6,6-trimethylen-9-phenyl-1,3-nonadien-8-inyl)-(1S)-cyclohexyl]-pentan-1-ol-tert.-butyldimethylsilylether (Diastereomer pol (12)) in 28 ml Dichlormethan und 0,78 ml Pyridin tropft man bei -70°C unter Argon 0,39 ml Diethylaminoschwefeltrifluorid und rührt 2 Stunden bei -70°C. Man läßt auf Raumtemperatur erwärmen, fügt vorsichtig eine 5%ige Natriumbicarbonatlösung zu, rührt 15 Minuten, verdünnt mit 200 ml Dichlormethan und wäscht mit je 30 ml Sole. Man trocknet über Natriumsulfat und dampft im Vakuum ein. Den Rückstand trennt man durch Chromatographie an Kieselgel. Mit Hexan/Essigsäurethylester (97+3 und 94+6) erhält man zunächst als unpolare Komponente 280 mg 5-[(E)-(2S)-2-((1E,3E)-(5S)-5-Acetoxy-6,6-trimethylen-9-phenyl-1,3-nonadien-8-inyl)-cyclohexyliden]-pentan-1-ol-tert.-butyldimethylsilylether, 800 mg Mischfraktionen und als polare Komponente 550 mg (5R)-5-Fluor-5-[cis-(2S)-2-((1E,3E)-(5S)-5-acetoxy-6,6-trimethylen-9-phenyl-1,3-nonadien-8-inyl)-(1S)-cyclohexyl]-pentan-1-ol-tert.-butyldimethylsilylether als farbloses Öl. IR-Spektrum des Olefins: (CHCL₃) 2930, 2858, 1729, 1248, 990, 837 cm⁻¹.

Zur Silyletherspaltung rührt man 220 mg des vorstehend hergestellten unpolaren Olefins in 12 ml Tetrahydrofuran mit 363 mg Tetrabutylammoniumfluorid 3 Stunden bei 24°C unter Argon. Anschließend verdünnt man mit Diethylether, wäscht dreimal mit Wasser, trocknet über Natriumsulfat und dampft im Vakuum ein. Den Rückstand chromatographiert man mit Hexan/Essigsäureethylester (8+2) an Kieselgel. Dabei erhält mam 140 mg des 1-Alkohols als farbloses Öl (polare Komponente). Als unpolare Komponente werden 50 mg des Z-konfigurierten Δ^{5.6}-Olefins hier abgetrennt. IR: 3430, 2925, 2850, 2220, 1735, 1665, 1240, 992 cm⁻¹.

Zur Oxidation der 1-Hydroxygruppe fügt man bei 0°C zu 350 mg des vorstehend hergestellten Alkohols in 25 ml Dichlormethan 1,6 g Collinsreagenz (Bis-pyridinchrom(VI)-oxid-Komplex; Tetrahedron Letters 1968, 3363) und rührt 10 Minuten bei 0°C. Anschließend verdünnt man mit einer Mischung aus Hexan/Diethylether (1+1), fügt Celite hinzu, filtriert, wäscht mit Hexan/Diethylether (1+1) und dampft im Vakuum ein. Der so erhaltene 1-Aldehyd wird sofort ohne weitere Reinigung verwendet.

Zu einer Lösung von 320 mg des vorstehend hergestellten Aldehyds in 6 ml Aceton tropft man unter Rühren bei -20°C 0,52 ml Jones-Reagenz (Chrom(VI)-oxid in H₂SO₄; J. Chem. Soc. 1953, 2555) und rührt 10 Minuten bei -20°C unter Argon. Anschließend fügt man 2,5 ml Isopropanol zu, rührt 5 Minuten, verdünnt mit 50 ml Diethylether, schüttelt zweimal mit Sole, trocknet über Natriumsulfat und dampft im Vakuum ein. Den Rückstand chromatographiert man an Kieselgel. Mit Hexan/Essigsäureethlester (3+2) erhält man 260 mg der 1-Carbonsäure als farbloses Öl.
IR: 3520, 2935, 2860, 1728, 1245, 992 cm⁻¹.

Zur Acetatverseifung fügt man zu 250 mg der vorstehend hergestellten Säure in 5 ml Methanol bei 25°C 5,3 ml einer 0,5 normalen Natronlauge und rührt 5 Stunden bei 25°C unter Argon. Anschließend säuert man mit 1 normaler Schwefelsäure auf pH 4-5 an. Man extrahiert mit Essigsäureethylester, wäscht zweimal mit Sole, trocknet über Natriumsulfat und dampft im Vakuum ein. Den Rückstand chromatographiert man an Kieselgel. Mit Essigsäureethylester erhält man 211 mg der Titelverbindung als farbloses Öl.
IR: 3400, 2930, 2855, 2220, 1708, 1599, 1490, 992 cm⁻¹.

Das Ausgangsmaterial für die obige Verbindung wird wie folgt hergestellt:

### 1a) 2-Oxo-3,3-trimethylen-6-phenyl-hex-5-in-phosphonsäuredimethylester

Zu einer Lösung von 4,1 g Diisopropylamin in 180 ml Tetrahydrofuran gibt man bei -30°C 250 ml einer 1,6 molaren Butyllithiumlösung in Hexan und anschließend eine Lösung von 20 g Cyclobutancarbonsäure in 20 ml Tetrahydrofuran. Man rührt noch 40 Minuten bei -10°C und tropft dann 43 g 1-Brom-3-phenyl-2-propin zu, rührt 16 Stunden bei 25°C und gießt auf 400 ml Eiswasser. Nach Ansäuern mit 2 N Salzsäure auf pH 4 wird mit Diethylether extrahiert, der Extrakt mit Sole gewaschen, über Magnesiumsulfat getrocknet und im Vakuum eingeengt Der Rückstand wird in 83 ml Methanol gelöst, mit 4,3 ml konz. Schwefelsäure versetzt und 6 Stunden am Rückfluß gekocht Man kühlt ab, rührt in Eiswasser ein und extrahiert mit Diethylether. Der Extrakt wird mit Wasser neutral gewaschen, mit Natriumsulfat getrocknet und der Diethylether im Vakuum eingedampft. Man erhält nach Destillation (Kp. 123-125°C bei 0,05 mm) 43 g 2,2-Trimethylen-5-phenyl-pent-4-insäuremethylester.

Zu einer Lösung von 59 g Methanphosphonsäuredimethylester in 700 ml Tetrahydrofuran tropft man bei -70°C 268 ml 1,6 M Butyllithiumlösung in Hexan. Nach 1 Stunde wird eine Lösung von 44 g des vorstehend hergestellten Esters in 120 ml Tetrahydrofuran zugetropft und weiter 5 Stunden bei -70°C gerührt. Anschließend wird mit 35 ml Essigsäureethylester versetzt und im Vakuum eingeengt. Der Rückstand wird in 100 ml Wasser gelöst und dreimal mit je 400 ml Dichlormethan extrahiert. Man trocknet über Natriumsulfat und dampft im Vakuum ein. Der Rückstand wird durch Säulenchromatographie an Kieselgel gereinigt. Mit Essigsäureethylester erhält man 43 g des Phosphonats als farblose Flüssigkeit.
IR: 3420, 2998, 2875, 1703, 1250 cm⁻¹.

### 1b) cis-(1S)-1-(Tert.-butyl-dimethylsilyloxy-methyl)-2(R)-2-formyl-cyclohexan

Zu einer Lösung von 500 g cis-(1S)-Hydroxymethyl-(2R)-acetoxymethyl-cyclohex-4-en (hergestellt beispielsweise nach K. Laumen et al., Tetrahedron Letters 26, 2073 (1985)) in 2400 ml Dimethylformamid fügt man bei 0°C 481 g Imidazol und 532 g tert.-Butyldimethylsilychlorid und rührt 20 Stunden bei 24°C. Man verdünnt mit Diethylether, schüttelt mit 500 ml einer 5%igen Schwefelsäure, wäscht mit Sole neutral, trocknet über Magnesiumsulfat und dampft im Vakuum ein. Den Rückstand chromatographiert man an Kieselgel. Mit Hexan/Essigsäureethylestergemischen erhält man 519 g cis-(1S)-tert.-Butyl-dimethylsilyloxymethyl-(2R)-acetoxymethyl-cyclohex-4-en.

Zur Hydrierung rührt man 379 g des vorstehend hergestellten Silylethers in 2400 ml Essigsäureethylester mit 20 g Palladium-10%ig auf Kohle unter einer Wasserstoffatmosphäre bei Raumtemperatur und Normaldruck. Nach 6 Stunden war keine Wasserstoffaufnahme erkennbar. Die Reaktionsmischung wurde filtriert und im Vakuum eingedampft. Dabei erhält man 359 g der hydrierten Verbindung.
[α]_{D} = -7,1° (c = 1,005, Aceton)

Zur Acetatabspaltung tropft man zu einer Lösung von 35 g des vorstehend hergestellten Silylethers in 450 ml Toluol 194 ml einer ca. 1,2 molaren Lösung von Diisobutylaluminiumhydrid in Toluol und rührt 15 Minuten bei -70°C. Anschließend tropft man 80 ml Isopropanol und dann 97 ml Wasser zu, rührt 2 Stunden bei 22°C, filtriert, wäscht mit Toluol und dampft im Vakuum ein. Den Rückstand reinigt man durch Chromatographie an Kieselgel. Mit Hexan/Essigsäureethylester (9+1) erhält man 22 g des Alkohols als farbloses Öl.
[α]_{D} = +3,5° (c = 1,350/ Aceton)
IR: 3420, 2925, 2858, 1465, 1255, 833 cm⁻¹.

Zur Überführung der Hydroxygruppe in die Formylgruppe tropft man bei -70°C 15,9 g Dimethylsulfoxid in 60 ml Dichlormethan zu einer Lösung von 12 g Oxalychlorid in 90 ml Dichlormethan und rührt 10 Minuten bei -60°C. Zu dieser Lösung fügt man bei -60°C eine Lösung von 19,5 g des vorstehend hergestellten Alkohols in 60 ml Dichlormethan, rührt 1,5 Stunden bei -60°C, tropft 30 ml Triethylamin zu und rührt 1,5 Stunden bei -50°C. Anschließend wird in 100 ml Eiswasser gegossen, zweimal mit je 50 ml Dichlormethan extrahiert, mit Wasser gewaschen, einmal mit 50 ml 5%iger Zitronensäure geschüttelt und zweimal mit Sole gewaschen. Man trocknet über Natriumsulfat und dampft im Vakuum ein. Man erhält 19,2 g des Aldehyds, der ohne weitere Reinigung verwendet wird.
IR: 2930, 2858, 2730, 1713, 840 cm⁻¹.

### 1c) 3-[cis-(1S)-1-Tert.-butyl-dimethylsilyloxymethyl)-(2S)-cyclohex-2-yl]-(2E)-propen-1-al

Zu einer Suspension von 4,42 g Lithiumchlorid in 300 ml Acetonitril tropft man unter Argon bei Raumtemperatur 20,7 ml Phosphonoessigsäuretriethylester und dann 13,9 ml Diazabicycloundecen (DBU) und rührt 15 Minuten. Anschließend tropft man eine Lösung von 19,12 g des unter 1b hergestellten Aldehyds in 40 ml Acetonitril zu, rührt 3 Stunden bei 24°C und verdünnt dann mit Diethylether. Man schüttelt nacheinander mit Wasser, 10%iger Schwefelsäure und Wasser, trocknet mit Natriumsulfat und dampft im Vakuum ein. Den Rückstand chromatographiert man mit Hexan/Diethylether (9+1) an Kieselgel. Dabei erhält man 17 g des α,β-ungesättigten Esters als farbloses Öl.
IR: 2930, 2838, 1706, 1648, 1270, 840 cm⁻¹.

Zur Reduktion der Estergruppe tropft man zu einer Lösung aus 17 g des vorstehend hergestellten Esters in 240 ml Toluol bei -70°C 86 ml einer 1,2 molaren Lösung von Diisobutylaluminiumhydrid in Toluol und rührt 30 Minuten bei -70°C. Anschließend tropft man 30 ml Isopropanol und dann 40 ml Wasser zu, rührt 2 Stunden bei 22°C, filtriert, wäscht mit Dichlormethan und dampft im Vakuum ein. Den Rückstand chromatographiert man mit Hexan/Essigsäureethyleser (4+1) an Kieselgel. Dabei erhält man 14,5 g des Allylalkohols als farbloses Öl.
IR: 3610, 3450, 2930, 2858, 1460, 838 cm⁻¹.

Eine Lösung von 14,4 g des vorstehend hergestellten Alkohols in 280 ml Toluol versetzt man mit 44 g Braunstein und rührt 5 Stunden bei 24°C. Anschließend wird filtriert, eingedampft und an Kieselgel chromatographiert. Mit Hexan/Diethylether (9+1) eluiert man 13,3 g des Aldehyds als farbloses Öl.
IR: 2930, 2860, 2740, 1685, 1630, 840 cm⁻¹.

### 1d) (5S)-5-Acetoxy-1-[cis-(1S)-1-hydroxymethyl)-(2S)-cyclohex-2-yl]-6,6-trimethylen-9-phenyl-(1E,3E)-1,3-nonadien-8-in

Zu einer Suspension von 5 g Natriumhydrid (60%ige Suspension in Öl) in 190 ml Dimethoxyethan tropft man bei 0°C eine Lösung aus 40,10 g 2-Oxo-3,3-trimethylen-6-phenyl-hex-5-in-phosphonsäuredimethylester in 290 ml Dimethoxyethan und rührt 1 Stunde bei 0°C. Anschließend tropft man eine Lösung des unter 1c) beschriebenen Aldehyds (30,75 g) in 350 ml Dimethoxyethan zu, rührt 1 Stunde bei 0°C, 4 Stunden bei 25°C und gießt dann auf 200 ml gesättigte Ammoniumchloridlösung. Man extrahiert dreimal mit Diethylether, wäscht die organische Phase mit Wasser, trocknet über Magnesiumsulfat und dampft im Vakuum ein. Den Rückstand reinigt man durch Säulenchromatographie an Kieselgel. Mit Hexan/Diethylether (9+1) erhält man 41 g des α,β-ungesättigten Ketons als farbloses Öl.
IR: 2925, 2858, 1673, 1625, 1590, 1001, 838 cm⁻¹.

Zur Reduktion der Ketogruppe fügt man zu einer Lösung von 40,5 g des vorstehend beschriebenen Ketons in 700 ml Methanol und 74 ml Tetrahydrofuran bei -60°C 4,75 g Ce(III)-chlorid·Heptahydrat, rührt 20 Minuten und versetzt dann portionsweise mit 5 g Natriumborhydrid. Man rührt 20 Minuten bei -60°C, versetzt mit 34 ml Aceton, rührt 15 Minuten, neutralisiert bei Raumtemperatur mit Eisessig und engt im Vakuum ein. Anschließend nimmt man den Rückstand in einer Diethylether/Wasser-Mischung auf, schüttelt die wässrige Phase mit Diethylether, wäscht die organische Phase mit Wasser neutral, trocknet über Natriumsulfat und dampft im Vakuum ein. Den Rückstand chromatographiert man mehrfach an Kieselgelsäulen. Mit Hexan/Essigsäureethylester (8+2) erhält man zunächst 11 g des unpolaren R-konfigurierten Alkohols (5R)-5-Hydroxy-1-[cis-(1S)-1-(tert.-butyl-dimethylsilyloxymethyl)-(2S)-cyclohex-2-yl]-6,6trimethylen-9-phenyl-(1E,3E)-1,3-nonadien-8-in sowie 18 g des polaren S-konfigurierten Alkohols (5S)-5-Hydroxy-1-[cis-(1S)-1-tert.-butyldimethylsilyoxymethyl)-(2S)-cyclohex-2-yl]-6,6-trimethylen-9-phenyl-(1E,3E)-1,3nonadien-8-in als farblose Öle.
IR (polarer Alkohol): 3530, 2925, 2853, 990, 838 cm⁻¹.

Zur Acetylierung fügt man zu einer Lösung von 17,8 g des vorstehend hergestellten polaren Alkohols in 60 ml Pyridin 30 ml Essigsäureanhydrid und rührt 16 Stunden bei Raumtemperatur. Anschließend engt man im Vakuum unter Zusatz von Toluol ein und chromatographiert den Rückstand an Kieselgel. Mit Hexan/Diethylether (9+1) erhält man 19,1 g des Acetats als farbloses Öl.
IR: 2925, 2852, 1727, 1245, 990, 838 cm⁻¹.

Zur Silyletherspaltung fügt man zu 19,1 g des vorstehend hergestellten Acetats in 480 ml Tetrahydrofuran bei 0°C 25 g Tetrabutylammoniumfluorid und rührt 3 Stunden bei 24°C. Anschließend verdünnt man mit Diethylether und wäscht dreimal mit Sole. Man trocknet über Magnesiumsulfat, dampft im Vakuum ein und chromatographiert den Rückstand an Kieselgel. Mit Hexan/Essigsäureethylester (7+3) eluiert man 14 g des Alkohols als farbloses Öl.
IR: 3450, 2930, 2858, 1729, 1245, 990 cm⁻¹.

### 1e) (5S)-5-Hydroxy-5-[cis-(2S)-2-((1E,3E)-(5S)-5-acetoxy-6,6-trimethylen-9-phenyl-1,3-nonadien-8-inyl)-(1S)-cyclohexyl]-pentan-1-ol-tert.-butyldimethyl silylether Diastereomer pol(12)

Zu einer Lösung von 8,95 g des vorstehend unter 1d) hergestellten Alkohols in 230 ml Dichlormethan fügt man bei 0°C 45 g Collins-Reagenz (Chromsäure-Pyridin-Komplex) und rührt 15 Minuten bei 0°C. Anschließend verdünnt man mit einer Mischung aus Hexan/Diethylether (2+1), fügt Celite hinzu, filtriert und dampft im Vakuum ein. Der so erhaltene Aldehyd wurde ohne weitere Reinigung verwendet (Rohausbeute 8,2 g).
IR: 2930, 2858, 2720, 1723, 1245, 990, 968 cm⁻¹.

Zur Grignardreaktion tropft man zu 5,76 g Magnesium bei 25°C unter Argon eine Lösung 26,7 g 4-Chlor-1-(tert.-butyldimethylsilyoxy)-butan in 24 ml Tetrahydrofuran, fügt ein Kristall Iod hinzu und rührt 30 Minuten bei 60°C. Anschließend verdünnt man mit 74 ml Tetrahydrofuran.
Zu 23 ml dieser Grignardlösung tropft man unter Argon bei -70°C die Lösung von 4,6 g des vorstehend hergestellten Aldehyds in 35 ml Tetrahydrofuran und rührt 30 Minuten bei -70°C. Man vesetzt mit gesättigter Ammoniumchloridlösung, extrahiert dreimal mit Diethylether, schüttelt die organische Phase mit Sole, trocknet über Natriumsulfat und dampft im Vakuum ein. Den Rückstand chromatographiert man an Kieselgel. Mit Hexan/Diethylether (9+1) erhält man zunächst 720 mg des 5R-konfigurierten diastereomeren Alkohols sowie als polarere Komponente 3,6 g des 5S-konfigurierten diastereomeren Alkohols (Titelverbindung).
IR: 3580, 2923, 2850, 1728, 1245, 990, 965, 835 cm⁻¹.

### Beispiel 2

### (5R)-5-Fluor-5-[cis-(2S)-2-((1E,3E)-(5S)-5-hydroxy-6,6-trimethylen-9-phenyl-1,3-nonadien-8-inyl)-(1S)-cyclohexyl]-pentansäure Diastereomer pol(12)

Zu einer Lösung von 540 mg des in Beispiel 1 hergestellten (5R)-5-Fluor-5-[cis-(2S)-2-((1E,3E)-(5S)-5-acetoxy-6,6-trimethylen-9-phenyl-1,3-nonadien-8-inyl)-(1S)-cyclohexyl]-pentan-1-ol-tert.-butyldimethylsilylethers in 24 ml Tetrahydrofuran fügt man 860 mg Tetrabutylammoniumfluorid und rührt 3 Stunden bei 24°C unter Argon. Anschließend verdünnt man mit Diethylether, wäscht dreimal mit Wasser, trocknet über Natriumsulfat und dampft im Vakuum ein. Den Rückstand chromatographiert man mit Hexan/Essigsäureethylester (7+3) an Kieselgel. Dabei erhält man 393 mg des 1-Alkohols als farbloses Öl.
IR: 3450, 2930, 2860, 1738, 1243, 992 cm⁻¹.

Zur Oxidation der 1-Hydroxygruppe fügt man bei 0°C zu 580 mg des vorstehend hergestellten Alkohols in 30 ml Dichlormethan 2,5 g Collins-Reagenz und rührt 10 Minuten bei 0°C. Anschließend verdünnt man mit einer Mischung aus Hexan/Diethylether (1+1), fügt Celite hinzu, filtriert, wäscht mit Hexan/Diethylether (1+1) und dampft im Vakuum ein. Der so erhaltene 1-Aldehyd wird sofort ohne weitere Reinigung verwendet.

Zu einer Lösung von 555 mg des vorstehend hergestellten Aldehyds in 10 ml Aceton tropft man unter Rühren bei -20°C 0,87 ml Jones-Reagenz und rührt 15 Minuten bei -20°C unter Argon. Anschließend fügt man 3,8 ml Isopropanol zu, rührt 5 Minuten, verdünnt mit 50 ml Diethylether, schüttelt zweimal mit Sole, trocknet über Natriumsulfat und dampft im Vakuum ein. Den Rückstand chromatographiert man an Kieselgel. Mit Hexan/Essigsäureethylester (4+1) erhält man 520 mg der 1-Carbonsäure als farbloses Öl.
IR: 3450, 2930, 2860, 1738, 1708, 1238, 992 cm⁻¹.

Zur Acetatverseifung fügt man zu 500 mg der vorstehend hergestellten Säure in 10 ml Methanol bei 25°C 10 ml einer 0,5 normalen Natronlauge und rührt 5 Stunden bei 25°C unter Argon. Anschließend säuert man mit 1 normaler Schwefelsäure auf pH 4-5 an. Man extrahiert mit Essigsäureethylester, wäscht zweimal mit Sole, trocknet über Natriumsulfat und dampft im Vakuum ein. Den Rückstand chromatographiert man an Kieselgel. Mit Essigsäureethylester/Hexan (4+1) erhält mam 420 mg der Titelverbindung als farbloses Öl.
IR: 3420, 2934, 2862, 2220, 1710, 1599, 993 cm⁻¹.

### Beispiel 3

### 5-[(E)-(2S)-2-((1E,3E)-(5R)-5-Hydroxy-6,6-trimethylen-9-phenyl-1,3-nonadien-8-inyl)-cyclohexyliden]-pentansäure Diastereomer unpol (12)

In Analogie zu Beispiel 1 erhält man aus dem in Beispiel 1d nach chromatographischer Trennung erhaltenen unpolaren R-konfigurierten Alkohols (5R)-5-Hydroxy-1-[cis-(1S)-1-(tert.-butyldimethylsilyloxymethyl)-(2S)-cyclohex-2-yl]-6,6-trimethylen-9-phenyl-(1E,3E)-1,3-nonadien-8-in die Titelverbindung als farbloses Öl.
IR: 3420, 2931, 2856, 2221, 1708, 1600, 1490, 991 cm⁻¹.

### Beispiel 4

### (5R)-5-Fluor-5-[cis-(2S)-2-((1E,3E)-(5R)-5-hydroxy-6,6-trimethylen-9-phenyl-1,3-nonadien-8-inyl)-(1S)-cyclohexyl]-pentansäure Diastereomer unpol (12)

In Analogie zu Beispiel 2 erhält man aus dem nach Beispiel 1 hergestellten (5R)-5-Fluor-5-[cis-(2S)-2-(1E,3E)-(5R)-5-acetoxy-6,6-trimethylen-9-phenyl-1,3-nonadien-8-inyl)-(1S)-cyclohexyl)-pentan-1-ol-tert.-butyldimethylsilylether die Titelverbindung als farbloses Öl.
IR: 3400, 2935, 2860, 2220, 1710, 1600, 992 cm⁻¹.

### Beispiel 5

### 5-[(E)-(2S)-2-((1E,3E)-(5R)-5-hydroxy-5-cyclohexyl-1,3-pentadienyl)-cyclohexyliden]-pentansäure Diasteromer pol (12)

In Analogie zu Beispiel 1 erhält man aus 1,5 g (5S)-5-Hydroxy-5-[cis-(2S)-2-((1E,3E)-(5R)-5-acetoxy-5-cyclohexyl-1,3-pentadien)-(1S)-cyclohexyl]-pentan-1-ol-tert.-butyldimethylsilylether (Diasteromer pol (12)) als unpolare Komponente 470 mg 5-[(E)-(2S)-2-((1E,3E)-(5R)-5-Acetoxy-cyclohexyl-1,3-pentadienyl)-cyclohexyliden]-pentan-1-ol-tert.-butyldimethylsilylether, 350 mg Mischfraktionen und als polarere Komponente 560 mg (5R)-5-Fluor-5-[cis-(2S)-2-((1E,3E)-(5R)-5-acetoxy-cyclohexyl-1,3-pentadienyl)-cyclohexyliden]pentan-1-ol-tert.-butyldimethylsilylether als farblose Ole.
IR Spektrum des Olefins: 2930, 2858, 1735, 1653, 1235, 1100, 990, 836 cm⁻¹.
IR-Spektrum des Fluorproduktes: 2930, 2858, 1737, 1238, 1102, 990, 938 cm⁻¹.

In Analogie zu der in Beispiel 1 beschriebenen Silyletherspaltung erhält man aus 460 mg des vorstehend hergestellten Olefins 270 mg des 1-Alkohols als farbloses Öl. Daneben trennt man hier 60 mg des isomeren Z-konfigurierten Δ^{5.6}-Olefins ab.
IR: 3450, 2923, 2858, 1733, 1236, 990, 972 cm⁻¹.

In Analogie zu der in Beispiel 1 beschriebenen Oxidation der 1-Hydroxygruppe erhält man aus 270 mg des vorstehend hergestellten 1-Alkohols 140 mg der 1-Carbonsäure als farbloses Öl.
IR: 3500, 2936, 2860, 1726, 1245, 991 cm⁻¹.

In Analogie zu der in Beispiel 1 beschriebenen Acetatverseifung erhält man 140 mg der vorstehend hergestellten Carbonsäure 118 mg der Titelverbindung als farbloses Öl.
IR: 3400, 2925, 2850, 1708, 1450, 990 cm⁻¹.

Das Ausgangsmaterial für die obige Titelverbindung wird wie folgt hergestellt:

### 5a) (5R)-5-Acetoxy-1-[cis-(1S)-1-hydroxymethyl)-(2S)-cyclohex-2-yl]-5-cyclo hexyl-(1E,3E)-pentadien Diastereomer pol (12)

Zu einer Suspension von 4,03 g Natriumhydrid (65%ige Suspension in Öl) in 195 ml Dimethoxyethan tropft man bei 0°C eine Lösung aus 26 g Dimethyl-(3-cyclohexyl-2-oxo-ethyl)-phosphonat in 283 ml Dimethoxyethan und rührt 1 Stunde bei 0°C. Anschließend tropft man eine Lösung des unter 1b beschriebenen Aldehyds in 470 ml Dimethoxyethan zu, rührt 1 Stunde bei 0°C, 4 Stunden bei 25°C und gießt dann auf gesättigte Ammoniumchloridlösung. Man extrahiert dreimal mit Diethylether, wäscht die organische Phase mit Wasser, trocknet über Magnesiumsulfat und dampft im Vakuum ein. Den Rückstand reinigt man durch Säulenchromatographie an Kieselgel. Mit Hexan/Diethylether (9+1) erhält man 35 g des ungesättigten Ketons als farbloses Öl.
IR: 2923, 2850, 1673, 1660, 1630, 1593, 1000, 835 cm⁻¹.

Zur Reduktion der Ketogruppe fügt man zu einer Lösung von 34,6 g des vorstehend beschriebenen Ketons in 885 ml Methanol und 89 ml Tetrahydrofuran bei -60°C 4,95 g Ce(III)-chlorid-Heptahydrat, rührt 15 Minuten und versetzt dann portionsweise mit 5 g Natriumborhydrid. Man rührt 15 Minuten bei -60°C, versetzt mit 35 ml Aceton, rührt 15 Minuten, neutralisiert bei Raumtemperatur mit Eisessig und engt im Vakuum ein. Anschließend nimmt man den Rückstand in einer Diethylether/Wasser-Mischung auf, schüttelt die wässrige Phase mit Diethylether, wäscht die organische Phase mit Wasser neutral, trocknet über Natriumsulfat und dampft im Vakuum ein. Den Rückstand chromatographiert man mehrfach an Kieselgel. Mit Hexan/Diethylether (97+3) erhält man zunächst 12 g des unpolaren S-konfigurierten Alkohols (5S)-5-Hydroxy-1-[cis-(1S)-1-(tert.-butyl-dimethylsilyloxymethyl)-(2S)-cyclohex-2-yl]-5-cyclohexyl-(1E,3E)-pentadien sowie 17 g des polaren R-konfigurierten Alkohols (5R)-5-Hydroxy-1-[cis-(1S)-1-(tert.-butyl-dimethylsilyloxymethyl)-(2S)-cyclohex-2-yl]-5-cyclohexyl-(1E,3E)-pentadien als farblose Öle.
IR: 3340, 2920, 2850, 990,838 cm⁻¹.

In Analogie zu der im Beispiel ld) beschriebenen Acetylierung erhält man aus 17 g des vorstehend beschriebenen polaren (5R)-konfigurierten Alkohols 17,4 g des Acetats als farbloses Öl.
IR: 2930, 2860, 1725, 1250, 992, 975, 840 cm⁻¹.

In Analogie zu der in Beispiel ld) beschriebenen Silyletherspaltung erhält man aus 13 g des vorstehend beschriebenen Acetats 9,84 g des Alkohols als farbloses Öl.
IR: 3620, 3450, 2932, 2860, 1725, 1250, 993, 945 cm⁻¹.

### 5b) (5S)-5-Hydroxy-5-[cis-(2S)-2-((1E,3E)-(5R)-5-acetoxy-5-cyclohexyl-1,3-pentadien)-(1S)-cyclohexyl]-pentan-1-ol-tert.-butyldimethylsilylether (Diastereomer pol (12))

In Analogie zu Beispiel le) erhält man aus 9,84 g des vorstehend unter 1d hergestellten Alkohols mit 77 g Collins-Reagenz den Aldehyd, der ohne weitere Reinigung verwendet wird.
In Analogie zu der in Beispiel le) beschriebenen Grignardreaktion erhält man aus 6,2 g des vorstehend hergestellten Aldehyds bei der chromatographischen Trennung zunächst 2,35 g des 5R-konfigurierten diastereomeren Alkohols sowie als polarere Komponente 5,15 g des 5S-konfigurierten diastereomeren Alkohols (Titelverbindung).
IR: 3570, 2922, 2852, 1729, 1244, 991, 836 cm⁻¹.

### Beispiel 6

### (5R)-5-Fluor-5-[cis-(2S)-2-((1E,3E)-(5R)-5-hydroxy-5-cyclohexyl-1,3-pentadienyl)-(1S)-cyclohexyl]-pentansäure Diastereomer pol (12)

In Analogie zu Beispiel 2 erhält man aus 560 mg des in Beispiel 5 hergestellten (5R)-5-Fluor-5-[cis-(2S)-2-((1E,3E)-(5R)-5-acetoxy-cyclohexyl-1,3-peniadienyl)-cyclohexyliden]-pentan-1-ol-tert.-butyldimethylsilylether und 1,04 g Tetrabutylammoniumfluorid 33 mg des 1-Alkohols als farbloses Öl.
IR: 3420, 2928, 2859, 1737, 1244, 992 cm⁻¹

In Analogie zu der in Beispiel 2 beschriebenen Oxidation des Alkohols zur 1-Carbonsäure erhält man aus 330 mg des vorstehend hergestellten Alkohols 230 mg der 1-Carbonsäure als farbloses Öl.
IR: 3500, 2930, 2860, 1725, 1248, 992 cm⁻¹.

In Analogie zu der in Beispiel 2 beschriebenen Acetatverseifung erhält man aus 230 mg der vorstehend hergestellten 1-Carbonsäure 218 mg der Titelverbindung als farbloses Öl.
IR: 3480, 2923, 2852, 1737, 1450, 1170, 990, 952, 920 cm⁻¹.

### Beispiel 7

### 5-[(E)-(2S)-2-((1E,3E)-(5S)-5-hydroxy-5-cyclohexyl-1,3-pentadienyl)-cyclohexyliden]-pentansäure Diastereomer unpol (12)

In Analogie zu Beispiel 1 und 3 erhält man aus dem in Beispiel 5a) nach chromatographischer Trennung erhaltenen unpolaren S-konfigurierten Alkohols (5S)-5-Hydroxy-1-[cis-(1S)-1-(tert.-butyl-dimethylsilyloxymethyl)-(2S).cyclohex-2-yl]-5-cyclo hexyl-(1E,3E)-pentadien die Titelverbindung als farbloses Öl.
IR: 3400, 2927, 2852, 1708, 1451, 991 cm⁻¹.

### Beispiel 8

### (5R)-5-Fluor-5-[cis-(2S)-2-((1E,3E)-(5S)-5-hydroxy-5-cyclohexyl-1,3-pentadienyl)-(1S)-cyclohexyl]-pentansäure Diastereomer unpol (12)

In Analogie zu Beispiel 2 und 6 erhält man aus dem nach Beispiel 5 hergestellten (5R)-5-Fluor-5-[cis-(2S)-2-((1E,3E)-(5S)-5-acetoxy-cyclohexyl-1,3-pentadienyl)-cyclohexyliden]-pentan-1-ol-tert.-butyldimethylsilylether die Titelverbindung als farbloses Öl
IR 3400, 2925, 2852, 1738, 1450, 1170, 990, 950, 920 cm⁻¹.

### Beispiel 9

### 5-[(E)-(2S)-2-((1E,3E)-(5S)-5-Hydroxy-6,6-trimethylen-9-phenyl-1,3-nonadien-8-inyl)-cyclohexyliden]-pentansäure-methylester Diastereomer pol(12)

Zu einer Lösung von 120 mg der nach Beispiel 1 hergestellten Säure in 4 ml Dichlormethan tropft man bei 0°C eine etherische Diazomethanlösung bis zur bleibenden Gelbfärbung und rührt 15 Minuten bei 0°C. Anschließend dampft man im Vakuum ein und reinigt den Rückstand durch Säulenchromatographie an Kieselgel. Mit Hexan/Essigsäureethylester (1+1) erhält man 116 mg der Titelverbindung als farbloses Öl.
IR:3472, 2928, 2853,2200, 1738, 1442, 1246, 1160, 992 cm⁻¹.

### Beispiel 10

### 5-[(E-(2S)-2-((1E,3E)-(5R)-5-hydroxy-5-cyclohexyl-1,3-pentadienyl)-cyclohexyliden]-pentansäure-methylester Diastereomer pol (12)

In Analogie zu Beispiel 9 erhält man aus 190 mg der nach Beispiel 5 hergestellten Säure 160 mg der Titelverbindung als farbloses Öl.
IR: 3470, 2930, 2855, 1739, 992 cm⁻¹.

### Beispiel 11

### 5-[(E-(2S)-2-((1E,3E)-(5S)-5-Hydroxy-6,6-trimethylen-9-phenyl-1,3-nonadien-8-inyl)-cyclohexyliden]-pentansäure-(3-hydroxypropylamid) Diastereomer pol (12)

Zu einer Lösung von 250 mg des nach Beispiel 9 hergestellten Methylesters in 8 ml Acetonitril fügt man 450 mg 3-Amino-1-propanol und rührt 24 Stunden bei 50°C und 24 Stunden bei 80°C. Anschließend wird im Vakuum eingedampft und der Rückstand durch Säulenchromatographie an Kieselgel gereinigt. Mit Dichlormethan/Methanol (9+1) erhält man 203 mg der Titelverbindung als farbloses Öl.
IR: 3340, 2928, 2828, 1651, 1530, 990 cm⁻¹.

### Beispiel 12

### Tris-(hydroxymethyl)-aminomethansalz von 5-[(E)-(2S)-2-((1E,3E)-(5S)-5-Hydroxy-6,6-trimethylen-9-phenyl-1,3-nonadien-8-inyl)-cyclohexyliden]-pentansäure Diastereomer pol (12)

Zu einer Lösung von 150 mg der nach Beispiel 1 hergestellten Carbonsäure in 24 ml Acetonitril fügt man bei 80°C 0,08 ml einer wässrigen Tris-(hydroxymethyl)aminomethan-Lösung (Herstellung: Man löst 8,225 g Trishydroxymethyl)aminomethan in 15 ml Wasser), rührt 1 Stunde bei 80°C, 1 Stunde bei 55°C, 3 Stunden bei 45°C und 60 Stunden bei 24°C. Man saugt die entstandenen Kristalle ab, wäscht mit etwas Acetonitril und trocknet die Kristalle bei 24°C im Vakuum. Dabei erhält man 140 mg der Titelverbindung als wachsartige Masse.
IR: 3320, 2922, 2852, 1550 (breit), 991 cm⁻¹.

### Beispiel 13

### 5-[(E)-(2S)-2-((1E,3E)-(5S)-5-Hydroxy-6,6-dimethyl-9-phenyl-1,3-nonadien-8-inyl)-cyclohexyliden]-pentansäure Diastereomer pol (12)

In Analogie zu Beispiel 1 erhält man aus 1,6 g (5S)-5-Hydroxy-5-[cis-(2S)-2-((1E,3E)-(5S)-5-acetoxy-6,6-dimethyl-9-phenyl-1,3-nonadien-8-inyl)-(1S)-cyclohexyl]-pentan-1-ol-tert.-butyldimethylsilylether (Diastereomer pol (12)) als unpolare Komponente 510 mg 5-[(E)-(2S)-2-((1E,3E)-(5S)-5-Acetoxy-6,6-dimethyl-9-phenyl-1,3-nonadien-8-inyl)-cyclohexyliden]-pentan-1-ol-tert.-butyldimethylsilylether 390 mg Mischfraktionen und als polare Komponente 410 mg (5R)-5-Fluor-5-[cis-(2S)-2-((1E,3E)-(5S)-5-acetoxy-6,6-dimethyl-9-phenyl-1,3-nonadien-8-inyl)-(1S)-cyclohexyl]-pentan-1-ol-tert.-butyldimethylsilylether als farbloses Öl.
IR-Spektrum des Olefins: 2931, 2860, 1730, 1250, 991 cm⁻¹.

In Analogie zu der in Beispiel 1 beschriebenen Silyletherspaltung erhält man aus 510 mg des vorstehend hergestellten Olefins 280 mg des 1-Alkohols als farbloses Öl. Daneben trennt man hier 80 mg des isomeren Z-konfigurierten Δ^{5.6}-Olefins ab.
IR: 3420, 2925, 2851, 2220, 1736, 1663, 1240, 992 cm⁻¹.

In Analogie zu der in Beispiel 1 beschriebenen Oxidation der 1-Hyroxygruppe erhält man aus 280 mg des vorstehend hergestellten 1-Alkohols 150 mg der 1-Carbonsäure als farbloses Öl.
IR: 3510, 2936, 2860, 1729, 1245, 992 cm⁻¹.

In Analogie zu der in Beispiel 1 beschriebenen Acetatverseifung erhält man aus 150 mg der vorstehend hergestellten Carbonsäure 122 mg der Titelverbindung als farbloses Öl.
IR: 3410, 2930, 2856, 2220, 1710, 1600, 1490, 991 cm⁻¹.

Das Ausgangsmaterial für die obige Titelverbindung wird in Analogie zu der in Beispiel la-le) beschriebenen Vorgehensweise hergestellt. Der für den Aufbau der Kette benötigte 2-Oxo-3,3-dimethyl-6-phenyl-hex-5-in-phosphonsäuredimethylester wird in Analogie zu Beispiel la) jedoch aus Isobuttersäure hergestellt.

### Beispiel 14

### (5R)-5-Fluor-5-[cis-(2S)-2-((1E,3E)-(5S)-5-Hydroxy-6,6-dimethyl-9-phenyl-1,3-nonadien-8-inyl)-(1S)-cyclohexyl]-pentansäure Diastereomer pol (12)

In Analogie zu Beispiel 2 erhält man aus 410 mg des in Beispiel 13 hergestellten (5R)-5-Fluor-5-[cis-(2S)-2-((1E,3E)-(5S)-5-acetoxy-6,6-dimethyl-9-phenyl-1,3-nonadien-8-inyl)-(1S)-cyclohexyl]-pentan-1-ol-tert.-butyldimethylsilylether und 810 mg Tetrabutylammoniumfluorid 260 mg des 1-Alkohols als Öl.
IR: 3450, 2930, 2860, 1740, 1245, 992 cm⁻¹.

In Analogie zu der in Beispiel 2 beschriebenen Oxidation des Alkohols zur 1-Carbonsäure erhält man aus 250 mg des vorstehend hergestellten Alkohols 180 mg der 1-Carbonsäure als farbloses Öl.

In Analogie zu der in Beispiel 2 beschriebenen Acetatverseifung erhält man aus 180 mg der vorstehend hergestellten 1-Carbonsäure 145 mg der Titelverbindung als farbloses Öl.
IR: 3430, 2935, 2863, 2200, 1710, 1599, 992 cm⁻¹.

## Patentansprüche

1. Leukotrien-B₄-Derivate der allgemeinen Formel I, 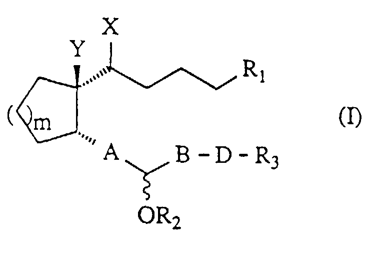 worin
R₁ CH₂OH, CH₃, CF₃, COOR₄, CONR₅R₆, und
R₂ H oder ein organischer Säurerest mit 1-15 C-Atomen darstellt,
R₃ H, gegebenenfalls einfach oder mehrfach substituiertes C₁-C₁₄-Alkyl, C₃-C₁₀-Cycloalkyl, gegebenenfalls unabhängig voneinander einfach oder mehrfach durch Halogen, Phenyl, C₁-C₄-Alkyl, C₁-C₄-Alkoxy, Fluormethyl, Chlormethyl, Trifluormethyl, Carbonyl, Carboxyl oder Hydroxy substituierter C₆-C₁₀-Arylrest oder ein 5-6 gliedriger aromatischer heterocyclischer Ring mit wenigstens 1 Heteroatom symbolisieren,
R₄ Wasserstoff, C₁-C₁₀ Alkyl, C₃-C₁₀ Cycloalkyl, gegebenenfalls durch 1-3 Halogen, Phenyl, C₁-C₄-Alkyl, C₁-C₄-Alkoxy, Fluormethyl, Chlormethyl, Trifluormethyl, Carboxyl oder Hydroxy substituierter C₆-C₁₀-Arylrest, CH₂-CO-(C₆-C₁₀) Aryl oder ein 5-6 gliedriger Ring mit wenigstens 1 Heteroatom bedeutet,
A eine trans, trans-CH=CH-CH=CH, eine -CH₂CH₂-CH=CH- oder eine Tetramethylengruppe,
B eine C₁-C₁₀- gerad- oder verzweigtkettige Alkylengruppe, die gegebenenfalls durch Fluor substituiert sein kann oder die Gruppe 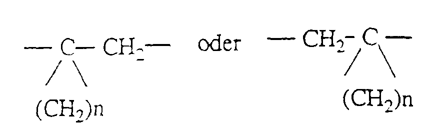 symbolisiert,
D eine Direktbindung, Sauerstoff, Schwefel, -C≡C-, -CH=CR₇, oder gemeinsam mit
B auch eine Direktbindung bedeuten können,
R₅ und R₆ gleich oder verschieden sind und H oder gegebenenfalls durch Hydroxygruppen substituiertes C₁-C₄-Alkyl oder R₆ H und R₅ C₁-C₁₅-Alkanoyl oder R₈SO₂- darstellen,
R₇ H, C₁-C₅-Alkyl, Chlor, Brom bedeutet,
R₈ die gleiche Bedeutung wie R₃ besitzt,
m 1-3 bedeutet
n 2-5 ist sowie, wenn R₄ Wasserstoff bedeutet, deren Salze mit physiologisch verträglichen Basen und deren Cyclodextrinclathrate.
X und Y eine direkte Bindung bedeutet, wobei das resultierende Olefin E oder Z konfiguriert sein kann oder X ein α- oder β-ständiges Fluoratom darstellt, und Y ein β-ständiges Wasserstoffatom bedeutet.

2. Pharmazeutische Präparate gekennzeichnet durch einen Gehalt an Leukotrien-B₄-Derivaten der allgemeinen Formel I gemäß Patentanspruch 1.

3. Verfahren zur Herstellung von Leukotrien-B₄-Derivaten der allgemeinen Formel I, gemäß Patentanspruch 1, dadurch gekennzeichnet, daß man einen Alkohol der Formel II 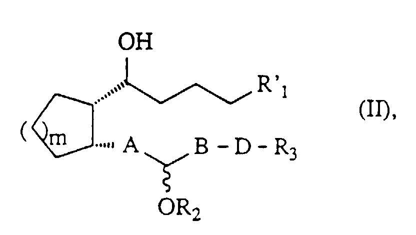 worin A, B, D, R₁, R₂ und R₃ die oben angegebene Bedeutung haben und R'₁ die gleiche Bedeutung wie R₁ besitzt oder die Gruppierung -CH₂OR₉ darstellt, worin R₉ einen leicht abspaltbaren Etherrest bedeutet, gegebenenfalls nach Schutz freier Hydroxygruppen in R₂ mit einem Wasserabspaltungsreagenz oder Fluorierungsreagenz der allgemeinen Formel III,
F₃SN(Alk)₃ (III)
wobei Alk -CH₃ oder -CH₂CH₃ darstellt, gegebenenfalls in Gegenwart einer Base umsetzt und gegebenenfalls anschließend in beliebiger Reihenfolge Isomere trennt, geschützte Hydroxygruppen freisetzt und/oder eine freie Hydroxygruppe verestert und/oder die 1-Hydroxygruppe zur Carbonsäure oxidiert und/oder Doppelbindungen hydriert und/oder eine veresterte Carboxylgruppe verseift und/oder reduziert und/oder eine Carboxylgruppe verestert und/oder eine freie Carboxylgruppe in ein Amid überführt oder eine Carboxylgruppe mit einer physiologisch verträglichen Base in ein Salz überführt.

## Claims

1. Leukotriene-B₄ derivatives of the general formula I 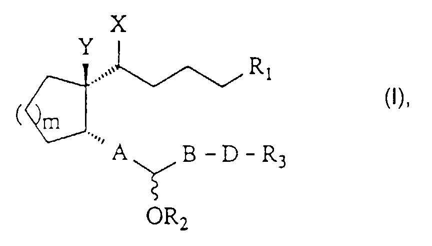 wherein
R₁ represents CH₂OH, CH₃, CF₃, COOR₄ or CONR₅R₆,
R₂ represents H or an organic acid radical having from 1 to 15 carbon atoms,
R₃ represents H, unsubstituted or mono- or poly-substituted C₁-C₁₄alkyl, C₃-C₁₀cycloalkyl; a C₆-C₁₀aryl radical that is unsubstituted or substituted by one or more substituents selected independently of one another from halogen, phenyl, C₁-C₄alkyl, C₁-C₄alkoxy, fluoromethyl, chloromethyl, trifluoromethyl, carbonyl, carboxy and hydroxy; or a 5- or 6-membered aromatic heterocyclic ring having at least 1 hetero atom,
R₄ represents hydrogen, C₁-C₁₀alkyl, C₃-C₁₀cycloalkyl; a C₆-C₁₀aryl radical that is unsubstituted or substituted from 1 to 3 times by halogen, phenyl, C₁-C₄alkyl, C₁-C₄alkoxy, fluoromethyl, chloromethyl, trifluoromethyl, carboxy or hydroxy; CH₂-CO-(C₆-C₁₀)aryl or a 5- or 6-membered ring having at least 1 hetero atom,
A represents a trans, trans-CH=CH-CH=CH- group, a -CH₂CH₂-CH=CH- group or a tetramethylene group,
B represents a straight-chain or branched-chain C₁-C₁₀alkylene group optionally substituted by fluorine or represents the group 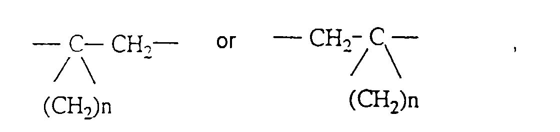
D represents a direct bond, oxygen, sulphur, -C≡C-, -CH=CR₇- or
B and D together can also represent a direct bond,
R₅ and R₆, which are the same or different, represent H or C₁-C₄alkyl optionally substituted by hydroxy groups, or R₆ is H and R₅ is C₁-C₁₅alkanoyl or R₈SO₂,
R₇ represents H, C₁-C₅alkyl, chlorine or bromine,
R₈ has the same meanings as R₃,
m is from 1 to 3,
n is from 2 to 5,
X and Y represent a direct bond, it being possible for the resulting olefin to be in the E- or Z-configuration, or X represents a fluorine atom in the α- or β-configuration and Y represents a hydrogen atom in the β-configuration,
and, when R₄ represents hydrogen, the salts thereof with physiologically tolerable bases and the cyclodextrin clathrates thereof.

2. Pharmaceutical preparations, characterised in that they comprise leukotriene-B₄ derivatives of the general formula I according to patent claim 1.

3. Process for the preparation of leukotriene-B₄ derivatives of the general formula I according to patent claim 1, characterised in that an alcohol of formula II 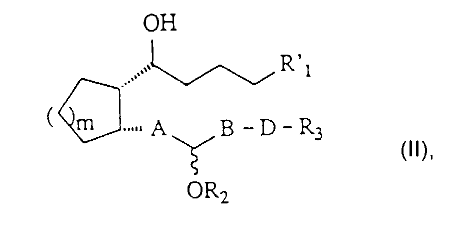 wherein A, B, D, R₁, R₂ and R₃ have the meanings mentioned above and R'₁ has the same meanings as R₁ or is the group -CH₂OR₉, wherein R₉ represents a readily removable ether radical, is reacted, optionally after protecting free hydroxy groups in R₂, with a water-removing reagent or with a fluorinating reagent of the general formula III,
F₃SN(Alk)₃ (III),
wherein Alk represents -CH₃ or -CH₂CH₃, optionally in the presence of a base, and then, where appropriate, in any sequence, isomers are separated, protected hydroxy groups are freed and/or a free hydroxy group is esterified and/or the 1-hydroxy group is oxidised to the carboxylic acid and/or double bonds are hydrogenated and/or an esterified carboxy group is hydrolysed and/or reduced and/or a carboxy group is esterified and/or a free carboxy group is converted into an amide or a carboxy group is converted into a salt with a physiologically tolerable base.

## Revendications

1. Dérivés du leucotriène B₄ de formule générale I, 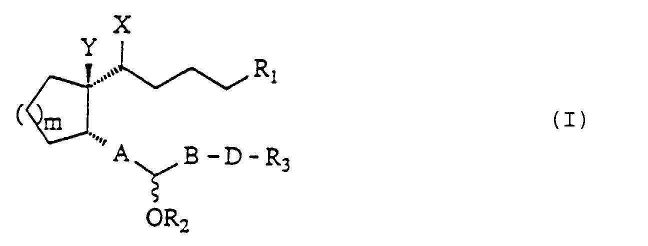 où
R₁ représente CH₂OH, CH₃, CF₃, COOR₄, CONR₅R₆, et
R₂ représente H ou un reste d'acide organique avec 1 à 15 atomes de carbone ;
R₃ représente H, un groupe alkyle en C₁-C₁₄ éventuellement substitué une ou plusieurs fois, un groupe cycloalkyle en C₃-C_{10,} un reste aryle en C₆-C₁₀ éventuellement substitué une ou plusieurs fois par des halogènes, des groupes phényle, alkyle en C₁-C₄, alcoxy en C₁-C₄, fluorométhyle, chlorométhyle, trifluorométhyle, carbonyle, carboxyle ou hydroxy, ou un héterocycle aromatique à 5 ou 6 chaînons avec au moins un héteroatome,
R₄ représente H, des groupes alkyle en C₁-C₁₀, cycloalkyle en C₃-C₁₀, un reste aryle en C₆-C₁₀ éventuellement substitué par de 1 à 3 atomes d'halogènes, des groupes phényle, alkyle en C₁-C₄, alcoxy en C₁-C₄, fluorométhyle, chlorométhyle, trifluoro-méthyle, carboxyle ou hydroxy, CH₂-CO-(aryle en C₆-C₁₀) ou un cycle à 5 ou 6 chaînons avec au moins un hétéroatome ;
A symbolise un groupe trans, trans-CH=CH-CH=CH, un groupe -CH₂CH₂-CH=CH- ou un groupe tétraméthylène ;
B symbolise un groupe alkyle en C₁-C₁₀ à chaîne linéaire ou ramifiée, qui peut éventuellement être substitué par le fluor, ou le groupe 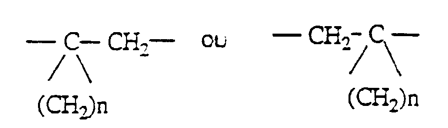
D peut représenter une liaison directe, l'oxygène, le soufre, -C≡C-, -CH=CR₇-, ou ensemble avec
B peut représenter aussi une liaison directe,
R₅ et R₆ sont identiques ou différents et représentent H, un groupe alkyle en C₁-C₄ éventuellement substitué par des groupes hydroxy, ou R₆ représente H et R₅ représente un groupe alcanoyle en C₁-C₁₅ ou R₈SO₂, éventuellement substitué par OH,
R₇ représente H, un groupe alkyle en C₁-C₅, des atome de chlore, de brome,
R₈ a la même signification que R₃,
m va de 1 à 3,
n va de 2 à 5 ainsi que, lorsque R₄ représente l'hydrogène, leurs sels avec des bases physiologiquement compatibles et leurs clathrates avec le cyclodextrine.
X et Y représentent une liaison directe, l'oléfine résultante pouvant être en configuration E ou Z, ou bien X représente un atome de fluor en position a ou β, et Y représente un atome d'hydrogène en position β.

2. Préparations pharmaceutiques caractérisées en ce qu'elle ont une teneur en dérivés du leucotriène B₄ de formule générale I, selon la revendication 1.

3. Procédé pour la préparation de dérivés du leucotriène B₄ de formule générale I, selon la revendication 1, caractérisé en ce que l'on fait réagir un alcool de formule II 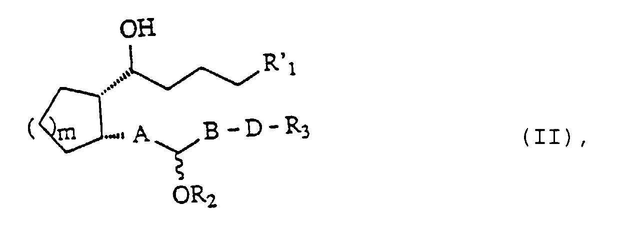 où A, B, D, R₁, R₂ et R₃ ont les significations données ci-dessus et R₁ a la même signification que R₁ ou représente les groupes -CH₂OR₉, où R₉ représente un reste éthéré facilement dissociable, éventuellement après protection des groupes hydroxys libres dans R₂ avec un réactif de dissociation de l'eau ou un réactif de fluoration de formule générale III,
F₃SN(alc)₃ (III)
où alc représente -CH₃ ou -CH₂CH₃, éventuellement en présence d'une base et éventuellement ensuite, on sépare les isomères dans un ordre quelconque, on libère les groupes hydroxy protégés et/ou on estérifie un groupe hydroxy libre et/ou on oxyde le groupe 1-hydroxy en acide carboxylique et/ou on hydrogène les doubles liaisons et/ou on saponifie un groupe carboxyle estérifié et/ou on réduit et/ou on estérifie un groupe carboxyle et/ou on transforme un groupe carboxyle libre en un amide ou un groupe carboxyle avec des bases physiologiquement compatibles, en un sel.
